(19) Europäisches Patentamt
European Patent Office
Office européen des brevets

(11) EP 1 575 575 B1

(12) **EUROPEAN PATENT SPECIFICATION**

(45) Date of publication and mention of the grant of the patent:
**19.05.2010 Bulletin 2010/20**

(21) Application number: **03810374.3**

(22) Date of filing: **31.10.2003**

(51) Int Cl.:
***A61K 31/04*** *(2006.01)* ***A61P 3/04*** *(2006.01)*

(86) International application number:
**PCT/DK2003/000742**

(87) International publication number:
**WO 2004/041256 (21.05.2004 Gazette 2004/21)**

(54) **SAFE CHEMICAL UNCOUPLERS FOR THE TREATMENT OF OBESITY**

SICHERE CHEMISCHE ENTKUPPLER ZUR BEHANDLUNG VON FETTSUCHT

DECOUPLEURS CHIMIQUES SURS POUR LE TRAITEMENT DE L'OBESITE

(84) Designated Contracting States:
**AT BE BG CH CY CZ DE DK EE ES FI FR GB GR HU IE IT LI LU MC NL PT RO SE SI SK TR**

(30) Priority: **08.11.2002 DK 200201719**
**14.05.2003 DK 200300734**
**04.06.2003 DK 200300827**

(43) Date of publication of application:
**21.09.2005 Bulletin 2005/38**

(73) Proprietor: **High Point Pharmaceuticals, LLC**
**High Point, NC 27265 (US)**

(72) Inventors:
- **HANSEN, Birgit, Sehested**
  **DK-3660 Stenløse (DK)**
- **TULLIN, Søren**
  **DK-2860 Søborg (DK)**
- **HANSEN, Thomas, Kruse**
  **DK-2730 Herlev (DK)**
- **COLDING-JØRGENSEN, Morten**
  **DK-2820 Gentofte (DK)**

(74) Representative: **Russell, Lindsey et al**
**Elkington and Fife LLP**
**Thavies Inn House**
**3-4 Holborn Circus**
**London EC1N 2HA (GB)**

(56) References cited:

| | |
|---|---|
| **WO-A-00/06143** | **WO-A-01/51923** |
| **GB-A- 2 284 153** | **GB-A- 2 312 375** |
| **US-A- 4 673 691** | **US-A- 5 240 962** |
| **US-A1- 2001 046 664** | **US-B1- 6 183 948** |

- **ROBERT M. DRUMMOND ET AL: "Release of Ca2+ from the sarcoplasmic reticulum increases mitochondrial (Ca2+) in rat pulmonary artery smooth muscle cells" JOURNAL OF PHYSIOLOGY, vol. 516, no. 1, 1999, pages 139-147, XP002271195**
- **ROLF ALTENBURGER ET AL: "Predictability of the toxicity of multiple chemical mixtures to vibrio fischeri: mixtures composed of similarly acting chemicals" ENVIRONMENTAL TOXICOLOGY AND CHEMISTRY, vol. 19, no. 9, 2000, pages 2341-2347, XP002271196**
- **WOO L W ET AL: "Steroidal and nonsteroidal sulfamates as potent inhibitors of steroid sulfatase." JOURNAL OF MEDICINAL CHEMISTRY. UNITED STATES 26 MAR 1998, vol. 41, no. 7, 26 March 1998 (1998-03-26), pages 1068-1083, XP002271197 ISSN: 0022-2623**
- **HUANG SHU-GUI: "Development of a high throughput screening assay for mitochondrial membrane potential in living cells." JOURNAL OF BIOMOLECULAR SCREENING: THE OFFICIAL JOURNAL OF THE SOCIETY FOR BIOMOLECULAR SCREENING. UNITED STATES AUG 2002, vol. 7, no. 4, August 2002 (2002-08), pages 383-389, XP002271198 ISSN: 1087-0571**
- **TERADA H ET AL: "Structural requirements of salicylanilides for uncoupling activity in mitochondria: quantitative analysis of structure-uncoupling relationships." BIOCHIMICA ET BIOPHYSICA ACTA. NETHERLANDS 7 DEC 1988, vol. 936, no. 3, 7 December 1988 (1988-12-07), pages 504-512, XP002271199 ISSN: 0006-3002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 575 575 B1

- **SABU KURUVILLA ET AL: "Effects of Minimally Toxic Levels of Carbonyl Cyanide P-(Trifluoromethoxy) Phenylhydrazone (FCCP), Elucidated through differential Gene Expression with Biochemical and Morphological Correlations" TOXICOLOGICAL SCIENCES, vol. 73, 2003, pages 348-361, XP002271200**

## Description

## FIELD OF THE INVENTION

This invention relates to chemical uncouplers with a broader safety window for use in treating obesity and, consequently, in the treatment of obesity related diseases and conditions such as atherosclerosis, hypertension, diabetes, especially type 2 diabetes (NIDDM (non-insulin dependent diabetes mellitus)), diabetic microvascular complication, impaired glucose tolerance, dyslipidemia, coronary heart disease, gallbladder disease, osteoarthritis as well as other conditions, such as diseases and disorders, which conditions are improved by an increase in mitochondrial respiration.

## BACKGROUND OF THE INVENTION

Obesity is a well-known risk factor for the development of many very common diseases such as atherosclerosis, hypertension, type 2 diabetes (non-insulin dependent diabetes mellitus (NIDDM)), dyslipidemia, coronary heart disease, and osteoarthritis and various malignancies. It also causes considerable problems through reduced motility and decreased quality of life. The incidence of obese people and thereby also these diseases is increasing throughout the entire industrialised world.

The term obesity implies an excess of adipose tissue. In this context obesity is best viewed as any degree of excess adiposity that imparts a health risk. The cut off between normal and obese individuals can only be approximated, but the health risk imparted by the obesity is probably a continuum with increasing adiposity. In the context of the present invention, individuals with a body mass index (BMI = body weight in kilograms divided by the square of the height in meters) above 25 are to be regarded as obese

Even mild obesity increases the risk for premature death and conditions such as diabetes, dyslipidemia, hypertension, atherosclerosis, gallbladder disease and certain types of cancer. In the industrialised western world the prevalence of obesity has increased significantly in the past few decades. Because of the high prevalence of obesity and its health consequences, its prevention and treatment should be a high public health priority.

Except for exercise, diet and food restriction, which is not feasible for a vast number of patients, no convincing treatment for reducing body weight effectively and acceptably currently exist. However, not only in view of the considerable problems directly related to obesity as described above, but also due to the important effect of obesity as a risk factor in serious and even mortal and common diseases, it is important to find pharmaceutical compounds which are useful in prevention and/or treatment of obesity.

When energy intake exceeds expenditure, the excess calories are stored predominately in adipose tissue, and if this net positive balance is prolonged, obesity results, i.e. there are two components to weight balance, and an abnormality on either side (intake or expenditure) can lead to obesity. This process may be counteracted by increasing the energy expenditure (for instance via exercise) or decreasing the energy intake (for instance by dieting). Pharmacological treatment available up to date only consists of Sibutramine (acting via serotonergic mechanisms, Abbott) and Orlistat (reducing fat uptake from the gut, Roche Pharm) neither reducing body weight effectively nor acceptably. There is therefore a need for pharmaceutical compounds which may be useful in prevention and/or treatment of obesity, for instance by increasing the energy expenditure or decreasing the energy intake.

One way of increasing energy expenditure is by increasing the metabolic rate. Oxidative phosphorylation in mitochondria, the energy from glucose metabolism and free fatty acids oxidation is used to drive the phosphorylation of ADP to ATP. When NADH and $FADH_2$ formed in the TCA cycle are oxidised back to $NAD^+$ and FAD respectively, protons are pumped out of the mitochondrial matrix. The resulting pH gradient (matrix pH~8 and outside pH~7) and potential (~-170 mV, inside negative) across the inner mitochondrial membrane constitute the electrochemical proton gradient. As the effect of a one-unit pH difference corresponds to a potential of 61.5mV, the electrochemical proton gradient exerts a protonmotive force of roughly -230 mV, which is the driving force for the mitochondrial ATP synthesis.

When the ATP consumption thus increases, the cells respond by increasing the ATP synthesis and consequently the inward flux of protons through the ATP synthase, the enzyme responsible for ATP synthesis and thereby the metabolic rate is increased. Chemical uncouplers are compounds, which can transport protons across membranes, and when protons are transported across the inner mitochondrial membrane, the ATP synthase is bypassed. At the (alkaline) matrix side the proton is released and the deprotonated uncoupler returns to the inter-membrane space where it picks up another proton. The cycling of the uncoupler (or ATP synthesis) and the resulting proton transport leads to an increased outward pumping of protons through an increased oxidation of NADH and $FADH_2$ by the respiration chain. The NADH concentration in the matrix will consequently drop. Since NADH feed-back inhibits three steps in the TCA cycle (NADH is the main regulator of the TCA cycle), the flux through the TCA cycle will increase. Hence, the metabolic rate will increase.

Compounds, such as chemical uncouplers, which act by increasing the metabolic rate may thus be useful for treating obesity, but also for treating other conditions such as atherosclerosis, hypertension, diabetes, especially type 2 diabetes (NIDDM (non-insulin dependent diabetes mellitus)), dyslipidemia, coronary heart disease, gallbladder disease,

osteoarthritis and various types of cancer such as endometrial, breast, prostate and colon cancers and the risk for premature death as well as other conditions, such as diseases and disorders, which conditions are improved by a reduced mitochondrial potential.

Furthermore, chemical uncouplers may reduce reactive oxygen species (ROS) that are assumed (De Grey et al, Eur J. Biochem 269, 1995 ff (2002)) to be involved in the aging process, in damage of heart tissue as well as neuronal tissue. It is therefore also possible that conditions affected by ROS may be reversed or halted by intervention by chemical uncouplers. Examples of such conditions include diabetic microvascular diseases in the retina, renal glomerulus and peripheral nerves cell.

The best known chemical uncoupler is 2,4-dinitrophenol (DNP), which has been shown to increase energy expenditure in humans as well as animals. The side effects at higher doses include increased perspiration, vasodilatation, skin rashes, cataracts, neuritis and death! Two fatalities amongst the first 100.000 persons treated with DNP, and the fact that the lowest dose, which could be lethal, was only twice the average dose giving a desired 50% increase in basal metabolic rate giving a very narrow safety window combined with other factors led to the removal of DNP from the market. Since then nobody have attempted to develop or market uncouplers for the treatment of obesity.

DNP is the best known chemical uncoupler; but many other compounds are known to induce uncoupling. DNP derivatives such as 4,6-dinitro-o-cresol (Victoria Yellow) and 2,4-dinitro-1-naphtol (Martius Yellow) as well as structural unrelated compounds such as 2,6-di*t*-butyl-4-(2',2'-dicyanovinyl)phenol) (SF6847) (also known as 2-(3,5-di-tert-butyl-4-hydroxybenzylidene)-malononitrile), carbonylcyanide m-chlorophenylhydrazone (CCCP) and carbonylcyanide ptrifluoromethoxy-phenylhydrazone (FCCP) (Miyoshi H et al. Quantitative releationship between protenophoric and uncoupling activities of analogs of SF6847 (2,6-di-t-butyl-4-(2',2'-dicyanovinyl)phenol), Biochimica et Biophysica Acta 891, 293-299 (1987)) are uncouplers.

Another class of chemical uncouplers is the salicylanilides of which S-13 is the most potent compound discovered so far (Terada H et al. Structural Requirements of Salicylanilides for Uncoupling Activity in Mitochondria Quantitative Analysis of Structure- Uncoupling Relationships, Biochimica et Biophysica Acta 936, 504-512 (1988)).

Goto K et al, Chem. Pharm. Bull. 44(3), 547-551 (1996) describes diethyl 4-[(4-bromo-2-cyanophenyl)carbamoyl]-benzylphosphonate for use as an LPL activator.

T. Shimokawa et al, Drug Development Research 51(1), 43-48 (2000) describes 5-chloro-N-(2-chloro-4-nitrophenyl)-2-hydroxy-3-methylbenzamide for use as a glucose uptake stimulator.

WO00/06143 to Texas Pharmaceuticals Inc. relates to a method for inducing intra-cellular hyperthermia comprising a step of administering a mitochondrial uncoupling agent, such as 2,4-dinitrophenol.

US 4,673,691 to Bachynsky relates to the use of 2,4-dinitrophenol for treating obesity.

SUMMARY OF THE INVENTION

The present invention provides chemical uncouplers as defined in claim 1 for use in enhancing mitochondrial respiration, which chemical uncouplers have an acceptably broad safety window making them useful for treating conditions benefiting from an enhancement of mitochondrial respiration, such as obesity, atherosclerosis, hypertension, diabetes, especially type 2 diabetes (NIDDM (non-insulin dependent diabetes mellitus)), dyslipidemia, coronary heart disease, gallbladder disease, osteoarthritis as well as other conditions, such as diseases and disorders, which conditions are improved by a reduced mitochondrial potential due to a saturation of the uncoupling and thereby the increase of the metabolism.

## DESCRIPTION OF THE FIGURES

Figure shows the curve for the stimulation of glucose utilisation caused by SF6847 calculated as a percentage of the stimulation of glucose utilisation caused by DNP according to Assay (I) and how the curve will look for a partial compound according to the present invention. $E_{max}$ is the highest level of stimulation that can be achieved by use if the test compound measured in percentages of the highest level of stimulation achieved by DNP. M is the molar concentration of test compound.

Figure shows how the calculation of $EC_{50}$ in Assay (I) is performed, exemplified by DNP and a test compound according to the present invention. $E_{max}$ is the highest stimulation of glucose utilisation achieved by the test compound and $EC_{50}$ is the concentration of test compound that gives a 50% stimulation.

Figure shows how the glucose utilisation depends on the concentration of the compound for 1) a compound with a decline in efficacy at concentrations slightly above the concentration at which $E_{max}$ is achieved ($CE_{max}$) and for 2) a compound with no decline in glucose utilisation at concentrations ranging from the concentration at which Emax is achieved ($CE_{max}$). See also Table 1.

## DEFINITIONS

The term "alkyl" as used herein, alone or in combination, refers to a straight or branched chain saturated monovalent hydrocarbon radical having from one to twelve carbon atoms, also denoted as $C_{1-12}$alkyl. Typical alkyl groups are alkyl groups with from one to eight or from one to six carbon atoms, also denoted as $C_{1-8}$-alkyl and $C_{1-6}$-alkyl respectively. Typical $C_{1-6}$-alkyl groups include, but are not limited to e.g. methyl, ethyl, n-propyl, isopropyl, n-butyl, sec-butyl, isobutyl, tert-butyl, n-pentyl, 2-methylbutyl, 3-methylbutyl, 4-methylpentyl, n-pentyl, n-hexyl, 1,1-dimethylpropyl, 1,2-dimethylpropyl, 2,2-dimethylpropyl (neopentyl), 1,2,2-trimethylpropyl and the like, while typical $C_{1-8}$-alkyl groups include the same groups as well as alkyl groups having seven or eight carbon atoms, such as heptyl, octyl, 2,2-dimethylhexyl and the like. The term "$C_{1-6}$-alkyl" as used herein also includes secondary $C_{3-6}$-alkyl and tertiary $C_{4-6}$-alkyl. The term "$C_{1-8}$-alkyl" as used herein also includes secondary $C_{3-8}$-alkyl and tertiary $C_{4-8}$-alkyl. The term "$C_{1-12}$-alkyl" as used herein also includes secondary $C_{3-12}$-alkyl and tertiary $C_{4-12}$-alkyl.

The term "alkenylene" as used herein, alone or in combination, refers to a straight or branched chain divalent hydrocarbon radical having from two to six carbon atoms and at least one carbon-carbon double bond, for example $C(_{3-5})$-alkenylene. Typical $C(_{3-5})$-alkenylene groups include, but are not limited to, propene-1,3-diyl, 1,3 butadiene-1,4-diyl, and the like. The term "conjugated alkenylene" as used herein, alone or in combination, refers to an alkenylene having consecutive double bonds, such as for instance 1,3 butadiene-1,4-diyl.

The term "cycloalkyl" as used herein, alone or in combination, refers to a nonaromatic carbocyclic monovalent hydrocarbon radical having from three to twelve carbon atoms, for example $C_{3-8}$-cycloalkyl. Such a ring may be optionally fused to one or more other cycloalkyl ring(s). Typical $C_{3-8}$-cycloalkyl groups include, but are not limited to, cyclopropyl, cyclobutyl, cyclopentyl, cyclohexyl, cycloheptyl, cyclooctyl and the like.

The term "aryl" as used herein, alone or in combination, refers to a carbocyclic aromatic ring radical or to a fused aromatic ring system radical, comprising at least one aromatic ring. Typical aryl groups include, but are not limited to, for example phenyl, biphenyl, naphtyl, and the like.

The term "heteroaryl", as used herein, alone or in combination, refers to an aromatic ring radical with for instance 5 to 7 member atoms, or to a fused aromatic ring system radical comprising at elast one heteroaromatic ring with for instance from 7 to 18 member atoms, containing one or more heteroatoms selected from nitrogen, oxygen, or sulfur heteroatoms, wherein N-oxides and sulfur monoxides and sulfur dioxides are permissible heteroaromatic substitutions; such as e.g. furanyl, thienyl, thiophenyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyridazinyl, pyrazinyl, pyrimidinyl, quinolinyl, isoquinolinyl, benzofuranyl, benzothiophenyl, indolyl, and indazolyl, and the like.

Examples of "aryl" and "heteroaryl" includes phenyl, biphenyl, indenyl, fluorene, naphthyl (1-naphthyl, 2-naphthyl), anthracenyl (1-anthracenyl, 2-anthracenyl, 3-anthracenyl), thienyl (2-thienyl, 3-thienyl), furanyl (2-furanyl, 3-furanyl), indolyl, oxadiazolyl, isoxazolyl, thiadiazolyl, oxatriazolyl, thiatriazolyl, quinazolinyl, fluorenyl, xanthenyl, isoindanyl, benzhydryl, acridinyl, thiazolyl, pyrrolyl (1-pyrrotyl, 2-pyrrolyl, 3-pyrrolyl), pyrazolyl (1-pyrazolyl, 3-pyrazolyl, 4-pyrazolyl, 5-pyrazolyl), imidazolyl (1-imidazolyl, 2-imidazolyl, 4-imidazolyl, 5-imidazolyl), triazolyl (1,2,3-triazol-1-yl, 1,2,3-triazol-4-yl 1,2,3-triazol-5-yl, 1,2,4-triazol-3-yl, 1,2,4-triazol-5-yl), oxazolyl (2-oxazolyl, 4-oxazolyl, 5-oxazolyl), isoxazolyl (isoxazo-3-yl, isoxazo-4-yl, isoxaz-5-yl), isothiazolyl (isothiazo-3-yl, isothiazo-4-yl, isothiaz-5-yl) thiazolyl (2-thiazolyl, 4-thiazolyl, 5-thiazolyl), pyridinyl (2-pyridinyl, 3-pyridinyl, 4-pyridinyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolinyl (2-quinolinyl, 3-quinolinyl, 4-quinolinyl, 5-quinolinyl, 6-quinolinyl, 7-quinolinyl, 8-quinolinyl), isoquinolinyl (1-isoquinolinyl, 3-isoquinolinyl, 4-isoquinolinyl, 5-isoquinolinyl, 6-isoquinolinyl, 7-isoquinolinyl, 8-isoquinolinyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydrobenzo[b]furanyl (2-(2,3-dihydrobenzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzob]furanyl), 7-(2,3-dihydro-benzo[b]furanyl)); benzo[b]thiophenyl (benzo[b]thiophen-2-yl, benzo[b]thiophen-3-yl, benzo[b]thiophen-4-yl, benzo[b]thiophen-5-yl, benzo[b]thiophen-6-yl, benzo[b]thiophen-7-yl), 2,3-dihydrobenzo[b]thiophenyl (2,3-dihydro-benzo[b]thiophen-2-yl, 2,3-dihydrobenzo[b]thiophen-3-yl, 2,3-dihydro-benzo[b]thiophen-4-yl, 2,3-dihydro-benzo[b]thiophen-5-yl, 2,3-dihydrobenzo[b]thiophen-6-yl, 2,3-dihydro-benzo[b]thiophen-7-yl), indolyl (1-indolyl, 2-indolyl, 3-indolyl, 4-indolyl, 5-indolyl, 6-indolyl, 7-indolyl), indazolyl (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (2-benzoxazolyl, 3-benzoxazolyl, 4-benzoxazolyl, 5-benzoxazolyl, 6-benzoxazolyl, 7-benzoxazolyl), benzothiazolyl (2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepinyl (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepinyl (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl), benzo[1,3]dioxole (2-benzo[1,3]dioxole, 4-benzo[1,3]dioxole, 5-benzo[1,3]dioxole, 6-benzo[1,3]dioxole, 7-benzo[1,3]dioxole), and tetrazolyl (5-tetrazolyl, N-tetrazolyl) as

well as partly or fully saturated analogues of the ring systems mentioned above.

The term "fused aromatic ring system" as used herein, alone or in combination, refers to a carbocyclic aromatic ring radical fused to another carbocyclic aromatic ring radical, the two having two atoms in common. Typical fused aromatic ring systems include, but are not limited to napthalene, quinoline, isoquinoline, indole, and isoindole.

A radical such as $C_{x-y}$-cycloalkyl-$C_{a-b}$-alkyl- shall designate that the radical's point of attachment is in part of the radical mentioned last.

As used herein, the term "optionally" means that the subsequently described event(s) may or may not occur, and includes both event(s) which occur and events that do not occur.

As used herein, the term "substituted" refers to substitution with the named substituent or substituents, multiple degrees of substitution being allowed unless otherwise stated.

Certain of the above defined terms may occur more than once in a given structural formulae, and upon such occurrence each term shall be defined independently of the other.

The term "nitro" shall mean the radical $-NO_2$.

The term "cyano" shall mean the radical -CN.

The term "halogen" shall mean -Cl, -F, -Br or -I.

As used herein, the term "solvate" is a complex of variable stoichiometry formed by a solute (in this case, a compound according to the present invention) and a solvent. Such solvents for the purpose of the invention may not interfere with the biological activity of the solute. Solvents may be, by way of example, water, ethanol, or acetic acid.

As used herein, the term "prodrug" includes biohydrolyzable amides and biohydrolyzable esters and also encompasses a) compounds in which the biohydrolyzable functionality in such a prodrug is encompassed in the compound according to the present invention, and b) compounds which may be oxidized or reduced biologically at a given functional group to yield drug substances according to the present invention. Examples of these functional groups include, but are not limited to, 1,4-dihydropyridine, N-alkylcarbonyl-1,4-dihydropyridine, 1,4-cyclohexadiene, tert-butyl, and the like.

A "therapeutically effective amount" of a compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The patient to be treated is preferably a mammal, in particular a human being.

The term "partial compound" as used herein is intended to indicate a compound with an $E_{max}$ as defined in assay I of less than 75% of that of FCCP, e.g. less than 70%, e.g. less than 65%, e.g. less than 60%, e.g. less than 55%, e.g. less than 50%, e.g. less than 45%, e.g. less than 40%, e.g. less than 35%, e.g. less than 30%, e.g. less than 25%, e.g. less than 20%, e.g. less than 15%, e.g. less than 10%.

## DESCRIPTION OF THE INVENTION

The present invention provides

The use of a compound of the general formula (III)

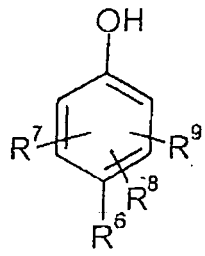

(III)

wherein

$R^6$ is halogen, -CHO, $-CO_2R^{43}$, $-COR^{43}$, $-SO_3H$, $-CCl_3$, $-CF_3$, -CN, $-CH=CH-R^{44}$, $-C(R^{44})(R^{45})$, $-SOR^{43}$, $-SO_2R^{43}$ or aryl substituted with from one to five substituents selected from halogen, -CHO, $-CO_2R^{43}$, $-COR^{43}$, $-SO_3H$, $-CCl_3$, $-CF_3$, -NO, $-NO_2$, -CN, $-CH=CH-R^{44}$, $-CH(R^{44})(R^{45})$, $-SOR^{43}$, $-SO_2R^{43}$, wherein

$R^{43}$ is hydrogen or alkyl; and

$R^{44}$ and $R^{45}$ independently of each other are halogen, -CHO, $-CO_2R^{46}$. $-COR^{46}$, $-SO_3H$, $-CCl_3$, $-CF_3$, -NO, $-NO_2$, -CN, $-SOR^{46}$, $-SO_2R^{46}$, wherein

$R^{46}$ is hydrogen, alkyl, or aryl;

$R^7$ is alkyl, halogen, alkyl-O-, alkyl-C(O)-, or alkyl-C(O)-O-; and

$R^8$ and $R^9$ independently of each other are hydrogen, alkyl, nitro, halogen, alkyl-O-, alkyl-C(O)-, alkyl-C(O)-O-, or aryl; or

$R^7$ and $R^8$ together form one of the diradicals

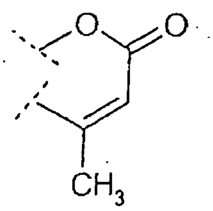 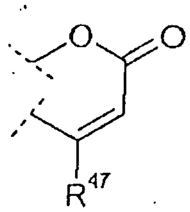 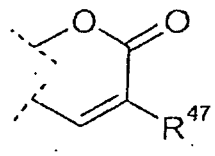 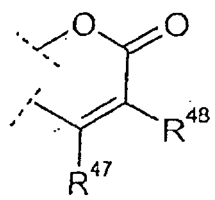 ,

wherein $R^{47}$ and $R^{48}$, independently of each other, are hydrogen, alkyl, nitro, halogen, alkyl-O-, alkyl-C(O)-, or alkyl-C(O)-O-,

wherein the two valence atoms in the diradical are attached to adjacent carbon atoms in the phenyl ring; and

$R^9$ is hydrogen, alkyl, nitro, halogen, alkyl-O-, or alkyl-C(O)-;

or a pharmaceutically acceptable salt, solvate or prodrug thereof,

for the manufacture of a medicament for treating a condition selected among: obesity, atherosclerosis, hypertension, diabetes, type 2 diabetes, impaired glucose tolerance, dyslipidemia, coronary heart disease, gallbladder disease, osteoarthritis, the aging process, damage to heart tissue, damage to endothelial cells, damage to neuronal tissue, Alzheimer's disease, cataract, diabetic microvascular diseases in the retina, renal glomerus and peripheral nerve cell apoptosis.

In one embodiment the compound has an $E_{max}$ in Assay (I), as described in the specification, of less than 75% of the $E_{max}$ of carbonylcyanide p-trifluoromethoxy-phenylhydrazone in Assay (I).

In a further embodiment, the compound has an $E_{max}$ in Assay (I), as described in the specification, of less than 70%, such as less than 65%, for instance less than 60%, such as less than 55%, for instance less than 50%, such as less than 45%, for instance less than 40%, such as less than 35%, for instance less than 30%, such as less then 25%, for instance less than 20%, such as less than 15%, for instance less than 10% of the $E_{max}$ of carbonylcyanide *p*-trifluoromethoxy-phenylhydrazone in Assay (I).

In a further embodiment the compound has an $E_{max}$ in Assay (I), as described in the specification, of less than 75% of the $E_{max}$ of 2,4-dinitrophenol (DNP) in Assay (I).

In a further embodiment, the compound has an $E_{max}$ in Assay (I), as described in the specification, of less than 70%, such as less than 65%, for instance less than 60%, such as less than 55%, for instance less than 50%, such as less than 45%, for instance less than 40%, such as less than 35%, for instance less than 30%, such as less then 25%, for instance less than 20%, such as less than 15%, for instance less than 10% of the $E_{max}$ of 2,4-dinitrophenol in Assay (I), or a pharmaceutically acceptable salt, solvate or prodrug thereof, for increasing mitochondrial respiration.

The $E_{max}$ of the compounds, FCCP and DNP is calculated as described below under the heading **"Assay (I):**

**Glucose utilisation in a human hepatocytes cell line (HEP-G2 cells)".**

In a further embodiment the compound has a slope calculated from the equation

$$X^n = (Y_2-Y_0)/ (Y_1-Y_0)$$

wherein

$Y_0$ is the degree of stimulation measured as counts per minute (cpm) in Assay (I) in control samples without added test compound,
$Y_1$ is the degree of stimulation measured as cpm in Assay (I) with added test compound in a concentration of either $EC_{50}/2$ or $EC_{50}/3$,
$Y_2$ is the degree of stimulation measured as cpm in Assay (I) with added test compound in concentration of either $2xEC_{50}$ or $3xEC_{50}$,
X is 2 or 3 depending on which concentration of $Y_1$ and $Y_2$ used, and n is the slope.

of a value equal to or less than the value for the slope calculated from the above equation with FCCP as test compound in Assay (I). In a further embodiment, said slope for said compound has a value of less than 95%, e.g. less than 90%, e.g. less than 80%, e.g. less than 70%, e.g. less than 50%, e.g. less than 40%, such as less than 20% of the value of said slope calculated for FCCP.

The equation should be understood such that when $Y_1$ is the degree of stimulation with added test compound in a concentration of $EC_{50}/2$, then $Y_2$ is the degree of stimulation with added test compound in concentration of $2xEC_{50}$, and X is 2, and when $Y_1$ is the degree of stimulation with added test compound in a concentration of $EC_{50}/3$, then $Y_2$ is the degree of stimulation with added test compound in a concentration of $3xEC_{50}$, and X is 3.

The values for use in the equation is calculated as described below under the heading: **"Assay (I): Glucose utilisation in a human hepatocytes cell line (HEP-G2 cells)".**

In a further embodiment, the slope is calculated by use of the computer software GraphPad Prism 3.0 (GraphPad software, Inc.).

In a further embodiment, the value for the slope calculated from the equation

$$X^n = (Y_2-Y_0)/ (Y_1-Y_0)$$

is significantly less than the value for the slope calculated with FCCP as test compound in Assay (I).

In a further embodiment the compound has a Hill slope, n, calculated as describes in Assay IV herein which is lower than or equal to the Hill slope calculated for FCCP. In a further embodiment, said Hill slope for said compound has a value of less than 95%, e.g. less than 90%, e.g. less than 80%, e.g. less than 70%, e.g. less than 50%, e.g. less than 40%, such as less than 20% of the value of said Hill slope calculated for FCCP.

In a further embodiment the value for the glucose utilisation caused by the compound in Assay (I) in concentrations of from $CE_{max}$ to ten times $CE_{max}$ does not fall significantly below the value of $E_{max}$ for the compound in Assay (I). $CE_{max}$ is calculated as described below under the heading: **"Assay (I):**

**Glucose utilisation in a human hepatocytes cell line (HEP-G2 cells)".**

Increasing the mitochondrial respiration as described above may take place *in vitro* or *in vivo,* for instance in an assay or in a subject.

More specifically the conditions treated according to the invention include type 2 diabetes (especially in obese patients), diabetes as a consequence of obesity, insulin resistance, hyperglycemia, prandial hyperglycemia, hyperinsulinemia, impaired glucose tolerance (IGT), impaired fasting glucose (IFG), increased hepatic glucose production, type 1 diabetes, LADA, pediatric diabetes, dyslipidemia (especially in obese patients), diabetic dyslipidemia, hyperlipidemia, hypertriglyceridemia, hyperlipoproteinemia,, micro-/macroalbuminuria, nephropathy, retinopathy, neuropathy, diabetic ulcers, cardiovascular diseases, arteriosclerosis, coronary artery disease, cardiac hypertrophy, myocardial ischemia, heart insufficiency, congestional heart failure, stroke, myocardial infarction, arrythmia, neurodegenerative and psychiatric disorders, Alzheimers disease, neuronal death, impaired cognitive function.

Uncouplers may also reduce insulin release from β-cells and may thus be useful in providing β-cell rest. Inducing β-cell rest may be useful In connection with β-cell transplantation, and it has also been described that inducing β-cell

rest may be useful in preventing diabetes.

The subject may be any mammal suffering from a condition benefiting from increased mitochondrial respiration. Such mammals may include, for instance, horses, cows, sheep, pigs, mice, rats, dogs, cats, primates such as chimpanzees, gorillas, rhesus monkeys, and, most preferably, humans.

Use of the compounds according to the present invention in the treatment of obesity may very likely reduce or eliminate the side effects such as irritation of the skin, glaucoma etc. known from treatment of obesity with DNP and other chemical uncouplers with narrow safety windows.

It is well-known that many compounds used to combat insects and parasites, i.e. insecticides and parasiticides, are chemical uncouplers. It is speculated that some of the adverse effects sometimes observed on animals or humans treated with or subjects exposed to such compounds may be caused by the fact that these compounds are not safe chemical uncouplers. It is thus believed that uncouplers according to the present invention could be used as insecticides or parasiticides with a lower risk to subjects treated with or exposed to the compounds.

In one embodiment of the present invention relating to compounds of formula III, $R^6$ represents halogen, -CHO, $-CO_2R^{43}$, $-COR^{43}$, $-SO_3H$, $-CCl_3$, $-CF_3$, -CN, $-CH=CH-R^{44}$, $-CH(R^{44})(R^{45})$, $-SOR^{43}$, $-SO_2R^{43}$. In particular, $R^{43}$ may represent hydrogen, or $R^{44}$ and $R^{45}$ independently of each other may represent halogen, -CHO, $-CO_2H$, -COH, $-SO_3H$, $-CCl_3$, $-CF_3$, -NO, $-NO_2$, -CN, -SOH, $-SO_2H$.

In any of the above mentioned embodiment relating to compounds of formula III, $R^6$ may represent cyano.

In any of the above mentioned embodiment relating to compounds of formula III, $R^7$ may represent alkyl, halogen, alkyl-O-, or alkyl-C(O)-. In particular, $R^7$ may represent alkyl, such as $C_{1-6}$-alkyl, e.g. methyl, ethyl, propyl, isopropyl, butyl, tert-butyl, pentyl, hexyl, or 1,1-dimethylpropyl, and in particular methyl. In particular, $R^7$ may represent alkyl-O-, such as $C_{1-6}$-alkyl-O-, e.g. wherein alkyl is selected from methyl, ethyl, propyl, isopropyl, butyl, tert-butyl, pentyl and hexyl, and in particular methyl. In particular, $R^7$ may represent alkyl-C(O)-, such as $C_{1-6}$-alkyl-C(O)-, e.g. wherein alkyl is selected from methyl, ethyl, propyl, isopropyl, butyl, tert-butyl, pentyl and hexyl, and in particular methyl. In particular, $R^7$ may represent alkyl-C(O)-O-, such as $C_{1-6}$alkyl-C(O)-O-, e.g. wherein alkyl is selected from methyl, ethyl, propyl, isopropyl, butyl, tert-butyl, pentyl and hexyl, and in particular methyl. $R^7$ and $R^8$ may also together form the diradical

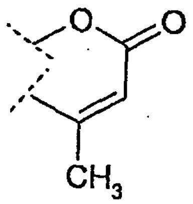 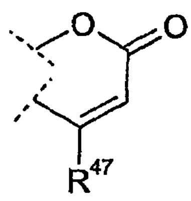 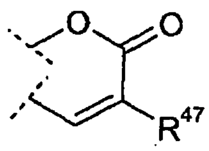 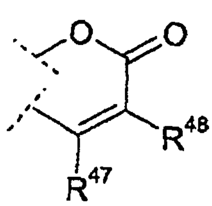 ,

wherein $R^{47}$ and $R^{48}$, independently of each other, are hydrogen, alkyl, nitro, halogen, alkyl-O-, alkyl-C(O)-, or alkyl-C(O)-O-. In particual, $R^{47}$ and/or $R^{48}$ may represent hydrogen.

In one embodiment relating to compounds of formula III, $R^8$ may represent hydrogen.

In any of the above mentioned embodiments relating to compounds of formula III, $R^9$. may represent hydrogen.

In a use according to the present invention, the compound may also be administered in combination with one or more further active substances in any suitable ratios. Such further active agents may be selected from antidiabetic agents, antihyperlipidemic agents, antiobesity agents, antihypertensive agents and agents for the treatment of complications resulting from or associated with diabetes.

Suitable antidiabetic agents include insulin, GLP-1 (glucagon like peptide-1) derivatives such as those disclosed in WO 98/08871 (Novo Nordisk A/S), which is incorporated herein by reference, as well as orally active hypoglycemic agents.

Suitable orally active hypoglycemic agents preferably include imidazolines, sulfonylureas, biguanides, meglitinides, oxadiazolidinedlones, thiazolidinediones, insulin sensitizers, α-glucosidase inhibitors, agents acting on the ATP-dependent potassium channel of the pancreatic β-cells eg potassium channel openers such as those disclosed in WO 97/26265, WO 99/03861 and WO 00/37474 (Novo Nordisk A/S) which are incorporated herein by reference, potassium channel openers, such as ormitiglinide, potassium channel blockers such as nateglinide or BTS-67582, glucagon antagonists such as those disclosed in WO 99/01423 and WO 00/39088 (Novo Nordisk A/S and Agouron Pharmaceuticals, Inc.), all of which are incorporated herein by reference, GLP-1 agonists such as those disclosed in WO 00/42026 (Novo Nordisk A/S and Agouron Pharmaceuticals, Inc.), which are incorporated herein by reference, DPP-IV (dipeptidyl peptidase-IV) inhibitors, PTPase (protein tyrosine phosphatase) inhibitors, glucokinase activators, such as those described in WO 02/08209 to Hoffmann La Roche, inhibitors of hepatic enzymes involved in stimulation of gluconeogenesis and/or glycogenolysis, glucose uptake modulators, GSK-3 (glycogen synthase kinase-3) inhibitors, compounds modifying the lipid metabolism such as antihyperlipidemic agents and antilipidemic agents, compounds lowering food intake, and

PPAR (peroxisome proliferator-activated receptor) and RXR (retinoid X receptor) agonists such as ALRT-268, LG-1268 or LG-1069.

In one embodiment of the uses of the present invention, the compound involved may be administered in combination with insulin or insulin analogues.

In one embodiment of the uses of the present invention, the compound involved may be administered in combination with a sulphonylurea eg tolbutamide, chlorpropamide, tolazamide, glibenclamide, glipizide, glimepiride, glicazide or glyburide.

In one embodiment of the uses of the present invention, the compound involved may be administered In combination with a biguanide eg metformin.

In one embodiment of the uses of the present invention, the compound involved may be administered in combination with a meglitinide eg repaglinide or senaglinide/nateglinide.

In one embodiment of the uses of the present invention, the compound involved may be administered in combination with a thiazolidinedione insulin sensitizer eg troglitazone, ciglitazone, pioglitazone, rosiglitazone, isaglitazone, darglitazone, englitazone, CS-011/CI-1037 or T 174 or the compounds disclosed in WO 97/41097 (DRF-2344), WO 97/41119, WO 97/41120, WO 00/41121 and WO 98/45292 (Dr. Reddy's Research Foundation), which are incorporated herein by reference.

In one embodiment of the uses of the present invention, the compound involved may be administered in combination with an insulin sensitizer eg such as GI 262570, YM-440, MCC-555, JTT-501, AR-H039242, KRP-297, GW-409544, CRE-16336, AR-H049020, LY510929, MBX-102, CLX-0940, GW-501516 or the compounds disclosed in WO 99/19313 (NN622/DRF-2725), WO 00/50414, WO 00/63191, WO 00/63192, WO 00/63193 (Dr. Reddy's Research Foundation) and WO 00/23425, WO 00/23415, WO 00/23451, WO 00/23445, WO 00/23417, WO 00/23416, WO 00/63153, WO 00/63196, WO 00/63209, WO 00/63190 and WO 00/63189 (Novo Nordisk A/S), which are incorporated herein by reference.

In one embodiment of the uses of the present invention, the compound involved may be administered in combination with an $\alpha$-glucosidase inhibitor eg voglibose, emiglitate, miglitol or acarbose.

In one embodiment of the uses of the present invention, the compound involved may be administered in combination with a glycogen phosphorylase inhibitor eg the compounds described In WO 97/09040 (Novo Nordisk A/S).

In one embodiment of the uses of the present invention, the compound involved may be administered in combination with a glucokinase activator.

In one embodiment of the uses of the present invention, the compound involved may be administered in combination with an agent acting on the ATP-dependent potassium channel of the pancreatic $\beta$-cells eg tolbutamide, glibenclamide, glipizide, glicazide, BTS-67582 or repaglinide.

In one embodiment of the uses of the present invention, the compound involved may be administered in combination with nateglinide.

In one embodiment of the uses of the present invention, the compound involved may be administered in combination with an antihyperlipidemic agent or a antllipidemic agent eg cholestyramine, colestipol, clofibrate, gemfibrozil, lovastatin; pravastatin, simvastatin, probucol or dextrothyroxine.

In one embodiment of the uses of the present invention, the compound involved may be administered in combination with more than one of the above-mentioned compounds eg in combination with metformin and a sulphonylurea such as glyburide; a sulphonylurea and acarbose; nateglinide and metformin; acarbose and metformin; a sulfonylurea, metformin and troglitazone; insulin and a sulfonylurea; insulin and metformin; insulin, metformin and a sulfonylurea; insulin and troglitazone; insulin and lovastatin; etc.

In one embodiment of the uses of the present invention, the compound involve may be administered in combination with one or more antiobesity agents or appetite regulating agents.

Such agents may be selected from the group consisting of CART (cocaine amphetamine regulated transcript) agonists, NPY (neuropeptide Y) antagonists, MC3 (melanocortin 3) agonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, $\beta$3 adrenergic agonists such as CL-316243, AJ-9677, GW-0604, LY362884, LY377267 or AZ-40140, MSH (melanocyte-stimulating hormone) agonists, MCH (melanocyte-concentrating hormone) antagonists, CCK (cholecystokinin) agonists, serotonin reuptake inhibitors (fluoxetine, seroxat or citalopram), serotonin and norepinephrine reuptake inhibitors, 5HT (serotonin) agonists, bombesin agonlsts, galanin antagonists, growth hormone, growth factors such as prolactin or placental lactogen, growth hormone releasing compounds, TRH (thyreotropin releasing hormone) agonists, UCP 2 or 3 (uncoupling protein 2 or 3) modulators, leptin agonists, DA (dopamine) agonists (bromocriptin, doprexin), lipase/amylase inhibitors, PPAR modulators, RXR modulators, TR $\beta$ agonists, adrenergic CNS stimulating agents, AGRP (agouti related protein) inhibitors, H3 histamine antagonists such as those disclosed in WO 00142023, WO 00/63208 and WO 00/64884, which are incorporated herein by reference, exendin-4, GLP-1 agonists and ciliary neurotrophic factor. Further antiobesity agents are bupropion (antidepressant), topiramate (anticonvulsant), ecopipam (dopamine D1/D5 antagonist), naltrexone (opioid antagonist), and

peptide $YY_{3-36}$ (Batterham et al, Nature 418, 650-654 (2002)).

In one embodiment, the antiobesity agent is leptin.

In one embodiment, the antiobesity agent is peptide $YY_{3-36}$.

In one embodiment, the antiobesity agent is a serotonin and norepinephrine reuptake inhibitor eg sibutramine.

In one embodiment, the antiobesity agent is a lipase inhibitor eg orlistat.

In one embodiment, the antiobesity agent is an adrenergic CNS stimulating agent eg dexamphetamine, amphetamine, phentermine, mazindol phendimetrazine, diethylpropion, fenfluramine or dexfenfluramine.

Furthermore, in the uses of the present invention, the compound involved may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-biockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the compounds according to the invention with diet and/or exercise, one or more of the above-mentioned compounds and optionally one or more other active substances are considered to be within the scope of the present invention.

The present invention also provides pharmaceutical compositions for use in treating a condition as defined in claim 1, the composition comprising as an active ingredient, at least one compound as defined above, preferably in a pharmacologically effective amount, more preferably in a therapeutically effective amount, together with one or more pharmaceutically acceptable carriers or excipients.

The pharmaceutical composition is preferably in unit dosage form, comprising from about 0.05 mg to about 1000 mg, preferably from about 0.1 mg to about 500 mg and especially preferred from about 0.5 mg to about 200 mg of a compound suitable for any of the uses described above.

## PHARMACEUTICAL COMPOSITIONS

The compounds for use according to the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 20th Edition, Gennaro, Ed., Mack Publishing Co., Easton, PA, 2000.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, preferably from about 0.1 to about 500 mg, and more preferred from about 0.5 mg to about

200 mg.

For parenteral routes such as intravenous, intrathecal, intramuscular and similar administration, typically doses are in the order of about half the dose employed for oral administration.

The present invention also encompasses pharmaceutically acceptable salts of the compounds suitable for use according to the present invention. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

Also intended as pharmaceutically acceptable acid addition salts are the hydrates which the present compounds are able to form.

The compounds for use according to the present invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which salts are generally prepared by reacting the free base with a suitable organic or inorganic acid or by reacting the acid with a suitable organic or inorganic base. When a compound for use according to the present invention contains a free base such salts are prepared in a conventional manner by treating a solution or suspension of the compound with a chemical equivalent of a pharmaceutically acceptable acid. When a compound for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds of the invention and these form a further aspect of the invention.

For parenteral administration, solutions of the compounds for use according to the present invention in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the compounds for use according to the present invention and the pharmaceutically acceptable carriers are then readily administered in a variety of dosage forms suitable for the disclosed routes of administration. The formulations may conveniently be presented in unit dosage form by methods known in the art of pharmacy.

Formulations of suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptabie excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay

disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsules wherein the active ingredient is mixed with water or an oil medium, for example peanut oil, liquid paraffin, or olive oil.

Aqueous suspensions may contain the compound for use according to the present invention in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising compounds for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

The compounds for use according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds for use according to the present invention may form solvates with water or common organic solvents. Such solvates are also encompassed within the scope of the invention.

Thus, in a further embodiment, there is provided a pharmaceutical composition for use according to the present invention comprising a compound of formula (III) as defined in claim 1, or a pharmaceutically acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet that may be prepared by conventional tabletting techniques may contain:

| | | |
|---|---|---|
| Core: | | |
| Example 19 | | 5.0 mg |
| Lactosum Ph. Eur. | | 67.8 mg |
| Cellulose, microcryst. (Avicel) | | 31.4 mg |
| Amberlite®iRP88* | | 1.0 mg |
| Magnesii stearas Ph. Eur. | | q.s. |
| Coating: | | |
| Hydroxypropyl methylcellulose | approx. | 9 mg |
| Mywacett 9-40 T** | approx. | 0.9 mg |

* Polacrillin potassium NF, tablet disintegrant, Rohm and Haas.

** Acylated monoglyceride used as plasticizer for film coating.

If desired, the pharmaceutical composition for use according to the present invention may comprise a compound for use according to the present invention in combination with further active substances such as those described in the foregoing. The compounds can be prepared readily according to the following general procedures (in which all variables are as defined before, unless so specified) using readily available starting materials, reagents and conventional synthesis procedures. In these reactions, It is also possible to make use of variants which are themselves known to those of ordinary skill in this art, but are not mentioned in greater detail.

**GENERAL PROCEDURES**

**General procedure (C)**

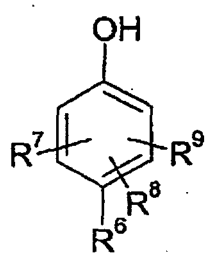

(III)

Compounds of the general formula (III), wherein $R^6$, $R^7$, $R^8$, and $R^9$ are as described above, are phenol derivatives with electro withdrawing groups that make the compounds of formula (III) more acidic than phenol.

The compounds of formula (III) may be prepared from the corresponding phenols by treatment with a mixture of sulphuric acid and nitric acid by methods which are known by those skilled in the art and described in Vogels *Textbook of practical organic chemistry.*

The following reference examples show compounds for use as a chemical uncoupler as described herein as well as comparative examples showing comparative compounds.

**HPLC-MS (Method A)**

The following instrumentation is used:

- Hewlett Packard series 1100 G1312A Bin Pump
- Hewlett Packard series 1100 Column compartment
- Hewlett Packard series 1100 G1315A DAD diode array detector
- Hewlett Packard series 1100 MSD
- Sedere 75 Evaporative Light Scattering detector

The instrument is controlled by HP Chemstation software.

The HPLC pump Is connected to two eluent reservoirs containing:

A: 0.01% TFA in water

B: 0.01% TFA in acetonitrile

The analysis is performed at 40°C by injecting an appropriate volume of the sample (preferably 1 µl) onto the column which is eluted with a gradient of acetonitrile.

The HPLC conditions, detector settings and mass spectrometer settings used are giving in the following table.

Column: Waters Xterra MS C-18 X 3 mm id 5 □m
Gradient: 5% - 100% acetonitrile linear during 7.5 min at 1.5ml/min
Detection: 210 nm (analogue output from DAD (diode array detector))
ELS (analogue output from ELS)
MS ionisation mode API-ES
Scan 100-1000 amu step 0.1 amu

After the DAD the flow is divided yielding approx 1 ml/min to the ELS and 0.5 ml/min to the MS.

## EXAMPLES

**Example 1** (General procedure (B))

4,4-Bis-(4-hydroxy-3-nitrophenyl)-valeric acid

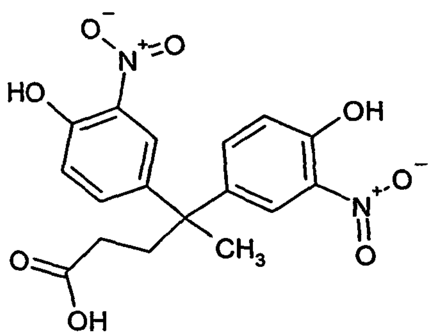

The title compound may be prepared according to General procedure B or purchased from Aldrich Chemical Company, Inc., catalogue number F209216.

**Example 2** (General procedure (A))

4-Methoxy-2-nitrophenol

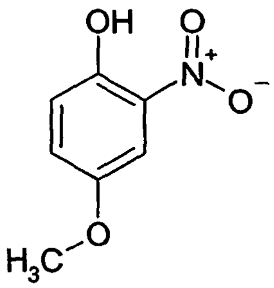

The title compound may be prepared according to General procedure A or purchased from Aldrich Chemical Company, Inc., catalogue number 1568-70-3.

**Example 3** (General procedure (A))

4-Hydroxy-3-nitroacetophenone

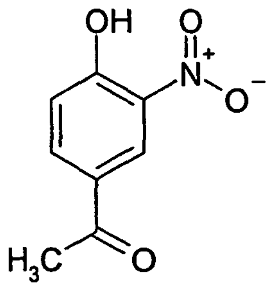

The title compound may be prepared according to General procedure A or purchased from Aldrich Chemical Company, Inc., catalogue number 6622-56-1.

**Example 4** (General procedure (A))

7-Hydroxy-4-methyl-8-nitro-chromen-2-one

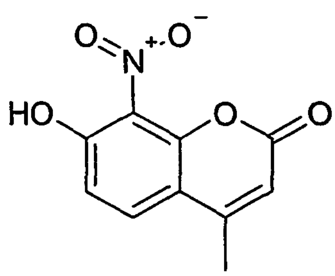

The title compound may be prepared according to General procedure A or purchased from Lancaster Synthesis Ltd., catalogue number F7896.

**Example 5** (General procedure (D))

3-Tert-butyl-5-chloro-N-(2-chloro-4-nitrophenyl)-2-hydroxy-6-methyl-benzamide

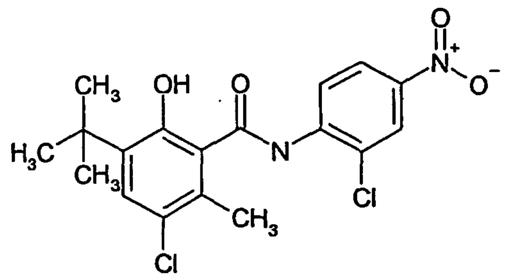

The title compound may be prepared according to General procedure D or purchased from Sigma-Aldrich Chemie GmbH, catalogue number R548758.

**Example 6** (General procedure (D))

N-1-[4-cyano-3-(trifluoromethyl)phenyl]-3,5-di(trifluoromethyl)benzamide

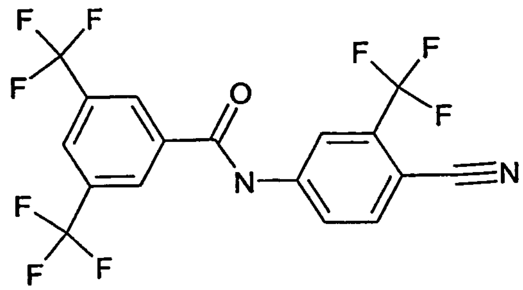

The title compound may be prepared according to General procedure D or purchased from Maybridge plc, catalogue number RDR03708.

**Example 7** (General procedure (D))

N-(4-cyanophenyl)benzamide

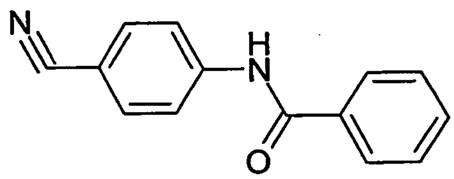

The title compound may be prepared according to General procedure D or purchased from Maybridge plc, catalogue number HTS05127.

**Example 8** (General procedure (D))

2'-Chloro-1-hydroxy-4'-nitro-2-naphthanilide

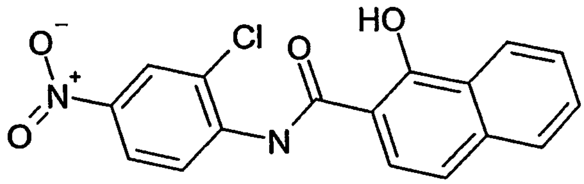

The title compound may be prepared according to General procedure D or purchased from Aldrich Chemical Company, Inc., catalogue number S62,938-3.

**Example 9** (General procedure (D))

N-(2-chloro-4-bromophenyl)-5-bromosalicylanilide

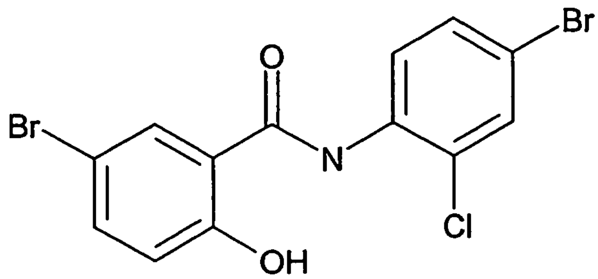

The title compound may be prepared according to General procedure D or purchased from Maybridge plc, catalogue number BTB 12821.

**Example 10** (General procedure (D))

N-(2-chloro-4-nitrophenyl)-3-tert-butyl-6-methylsalicylanilide

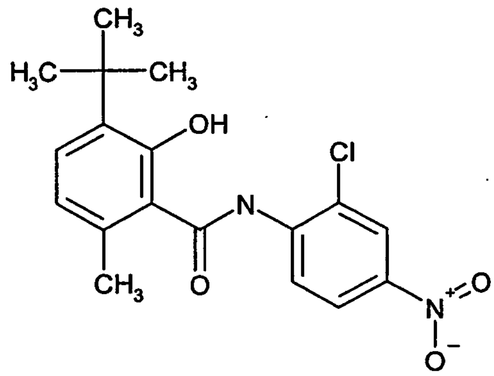

3-tert-Butyl-6-methylsalicylic acid (5.0 g, 24 mmol) and 2-chlor-4-nitroanilin (4.1 g; 24 mmol) were dissolved in chlorobenzene (140 ml). Phophoroxychloride (0.63 ml) was added and the mixture was refluxed 4.5 hours under nitrogen atmosphere. The solvent was removed in vacuo and the residue crystallized from acetic acid yielding 6.3 g of the title compound.

1H-NMR: (CDCl3, 400 MHz): 1.43 (s, 9H); 2.65 (s, 3H); 6.76 (d, 1H); 7.34 (d, 1H); 8.22 (dd, 1H); 8.42 (d, 1H); 8.47 (s (br); 1H); 8.86 (d, 1H); 10.3 (s (br); 1H).

**Example 11** (General procedure (E))

(3,5-Di-tert-butyl-4-hydroxybenzyl)triphenylphosphonium bromide

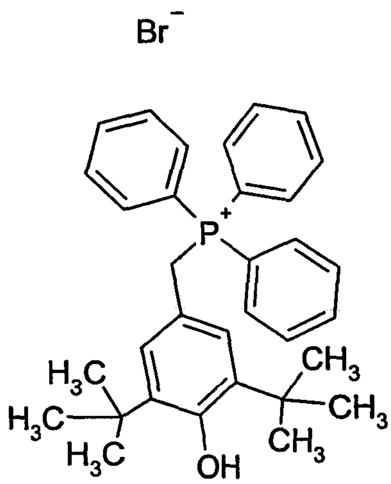

The title compound may be prepared according to General procedure E or purchased from Sigma-Aldrich Chemie GmbH, catalogue number S545813.

**Example 12** (General procedure (E))

(3,5-Di-tert-butyl-4-hydroxybenzyl)tricyclohexylphosphonium bromide

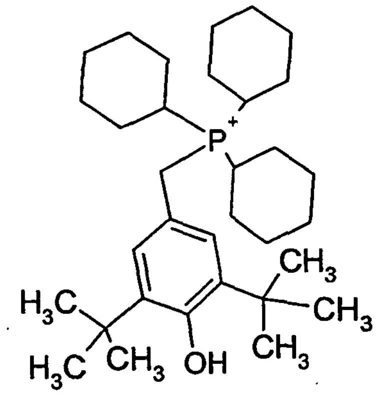

The title compound may be prepared according to General procedure E. 2,6-Di-*tert*-butyl-4-methylphenol is treated with 1 equivalent of N-bromo-succinimide in tetrachloromethane under reflux for 1 hour in the presence of 0.1 equivalent of benzoylperoxide. After evaporation of the solvent, 2,6-di-*tert*-butyl-4-bromomethylphenol is isolated using gradient elution on silica gel with heptane/methylene chloride as eluent. The crude 2,6-di-*tert*-butyl-4-bromomethylphenol (1 equivalent) is dissolved in dry diethyl ether (10% solution) and treated with a 10% solution of trialkyl- or triarylphosphine in diethyl ether. After 1 hour of stirring, crystalline material is filtered off. LCMS m/z: 499.5 $(M+H)^+$

**Example 13** (General procedure (E))

(3,5-Di-tert-butyl-4-hydroxybenzyl)tributylphosphonium bromide

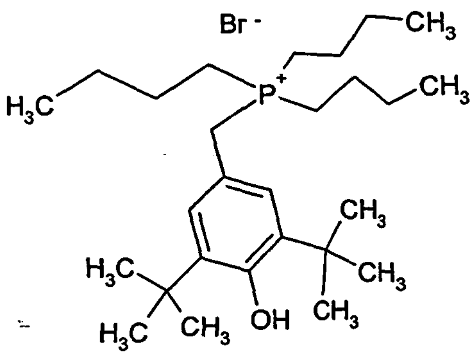

The title compound may be prepared according to General procedure E. 2,6-Di-*tert*-butyl-4-methylphenol is treated with 1equivalent of N-bromo-succinimide in tetrachloromethane under reflux for 1 hour in the presence of 0.1 equivalent of benzoylperoxide. After evaporation of the solvent, 2,6-di-*tert*-butyl-4-bromomethylphenol is isolated using gradient elution on silica gel with heptane/methylene chloride as eluent. The crude 2,6-di-*tert*-butyl-4-bromomethylphenol (1 equivalent) is dissolved in dry diethyl ether (10% solution) and treated with a 10% solution of tributylphosphine in diethyl ether. After 1 hour of stirring, crystalline material is filtered off. LCMS m/z: 421.3 $(M+H)^+$

**Example 14** (General procedure (E))

(3,5-Di-tert-butyl-4-hydroxybenzyl)trioctylphosphonium bromide

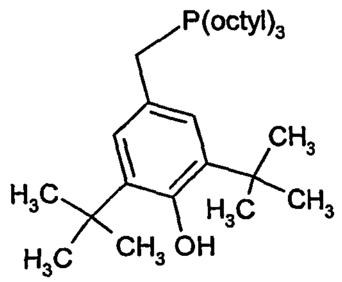

The title compound may be prepared according to General procedure E. 2,6-Di-*tert*-butyl-4-methylphenol is treated with 1 equivalent of N-bromo-succinimide in tetrachloromethane under reflux for 1 hour in the presence of 0.1 equivalent of benzoylperoxide. After evaporation of the solvent, 2,6-di-*tert*-butyl-4-bromomethylphenol is isolated using gradient elution on silica gel with heptane/methylene chloride as eluent. The crude 2,6-di-*tert*-butyl-4-bromomethylphenol (1 equivalent) is dissolved in dry diethyl ether (10% solution) and treated with a 10% solution of trioctylphosphine in diethyl ether. After 1 hour of stirring, the ether is decanted off, the oil triturated with ether and the remaining oil is dried *in vacuo.* LCMS m/z: 591.0 $(M+H)^+$

**Example 15** (General procedure (F))

2-Cyano-3-(3,5-di-tert-butyl-4-hydroxyphenyl)-acrylic acid ethyl ester

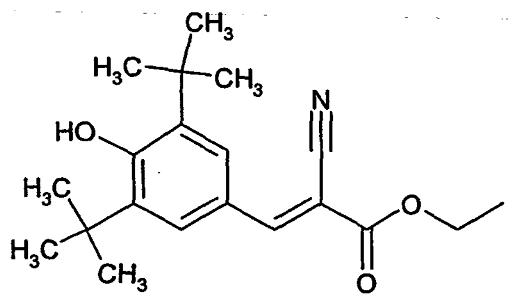

The title compound may be prepared according to General procedure F or purchased from Sigma-Aldrich Chemie GmbH, catalogue number S482102.

**Example 16** (General procedure (F))

2-(3,5-Di-tert-butyl-4-hydroxy-benzylidene)-malonic acid diethyl ester

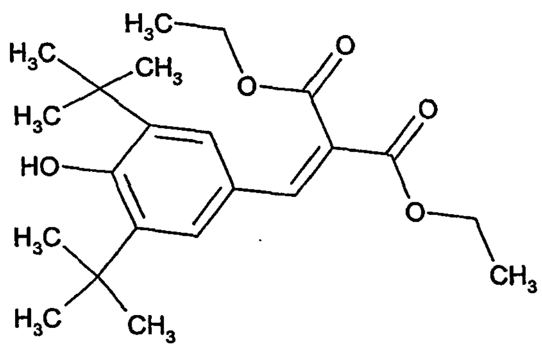

The title compound may be prepared according to General procedure F or purchased from Sigma-Aldrich Chemie GmbH, catalogue number S482196.

**Example 17** (General procedure (F))

2-Amino-S-[(3,5-di-tert-butyl-4-hydroxybenzylidone)-amino]-but-2-enedinitrile

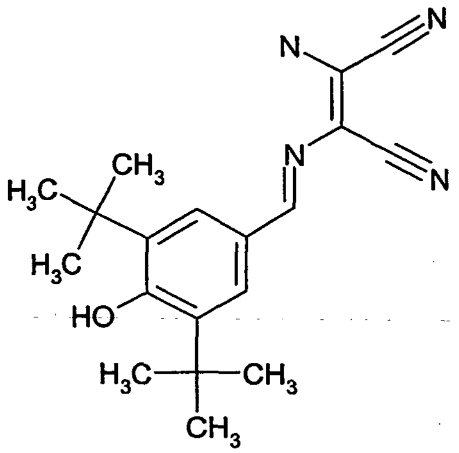

The title compound may be purchased from Menai, catalogue number DW 150.

**Example 18** (General procedure (F))

2-(3,5-Di-tert-butyl-4-hydroxy-benzylidene)-indan-1,3-dione

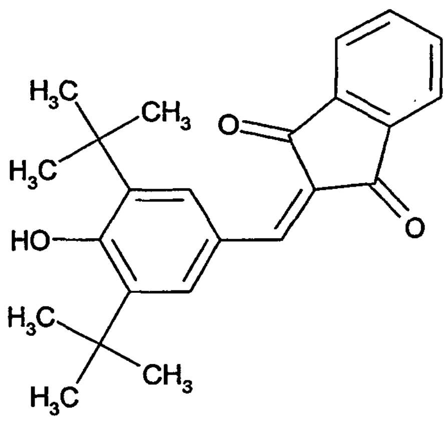

The title compound may be prepared according to General procedure F or purchased from Maybridge plc, catalogue number LJ 902.

**Example 19** (General procedure (G))

2-[[2-(4-Chlorophenyl)-1H-indol-3-yl]methylene]malononitrile

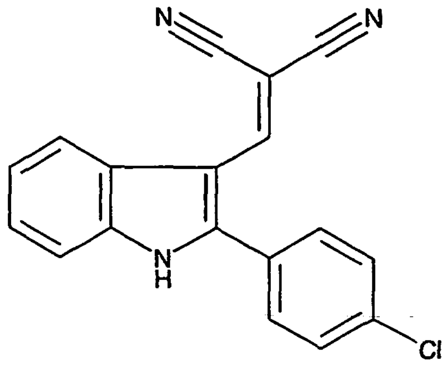

The title compound may be prepared according to General procedure G or purchased from Maybridge plc, catalogue number SEW 03041.

**Example 20** (General procedure (G))

2-(4-Chlorophenyl)-indole

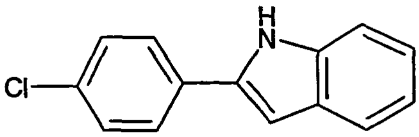

The title compound may be prepared according to General procedure G or purchased from Maybridge plc, catalogue number RDR 01154.

**Example 21**

N-(2,4,5-trichlorophenyl)salicylanilide

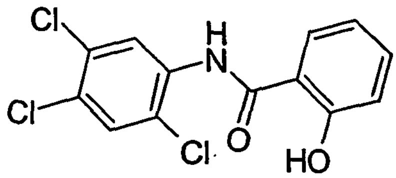

The title compound may purchased from Maybridge plc, catalogue number RDR 01398.

**Example 22** (General procedure (G))

2,3-Dimethyl-5-cyano-7-ethylindole

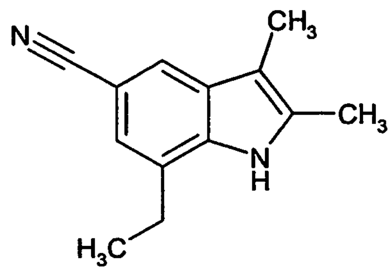

The title compound may be purchased from Aldrich Chemical Company, Inc., catalogue number 30,205-8.

**Example 23** (General procedure (G))

4-Bromo-2-(4-chlorophenyl)-5-trifluoromethyl-1H-pyrrole-3-carbonitrile

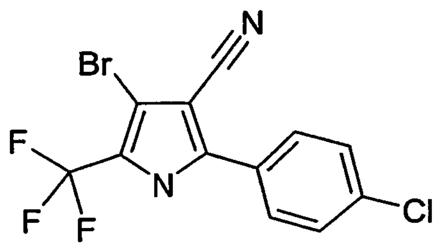

This compound was prepared as described in Kameshwaran, Synthesis 5, 530 (1997).

**Example 24**

N-(3-Cyano-4-phenylsulfanyl-phenyl)-3-trifluoromethyl-benzamide

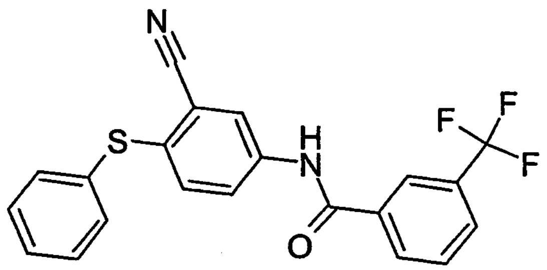

This compound is commercially available from Bionet (cat nr. 11K-627S).

**Example 25**

2,6-Di-*t*-butyl-4-(2',2'-dicyanovinyl)phenol

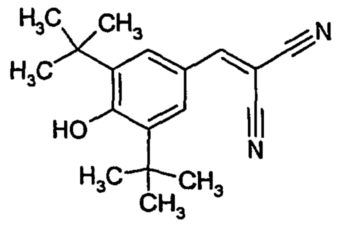

The title compound may be prepared according to General procedure F or purchased from Biomol research Laboratories Inc., catalogue number EI-215.

**Comparative Example 26**

2,4-Dinitrophenol

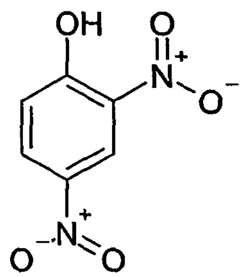

Available from Sigma-Aldrich Chemie GmbH, catalogue number D-7004.

**Comparative Example 27**

Carbonylcyanide p-trifluoromethoxy-phenylhydrazone

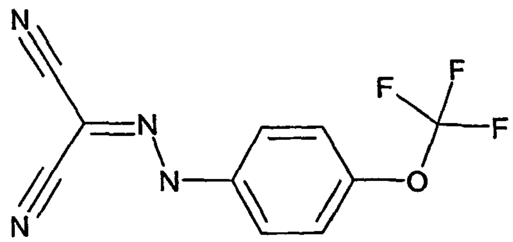

Available from Sigma-Aldrich Chemie GmbH, catalogue number F-86,184-7.

New examples for patent 6433.0000.

**Example 28**.(General procedure (G))

2-(5,7-Dimethyl-1H-indol-3-ylmethylene)-malononitrile

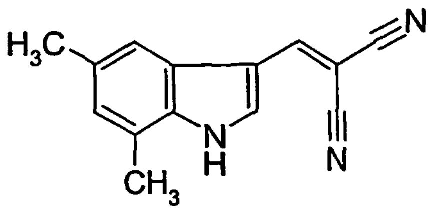

This compound is commercially available from Salor, R43,763-8.

**Example 29.**(General procedure (G))

2-(5-Bromo-1H-indol-3-ylmethylene)-malononitrile

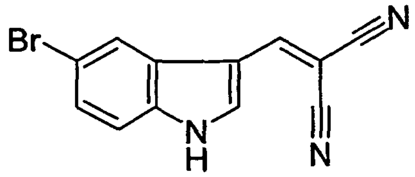

This compound is commercially available from Salor, R55,004-3.

**Example 30**.(General procedure (G))

2-((5-Chloro-1H-indol-3-yl)methylene)malononitrile

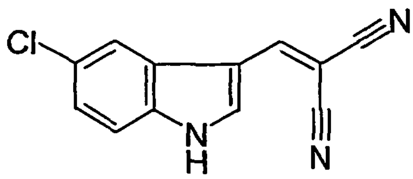

This compound is commercially available from Salor, R94,793-8.

**Example 31**.(General procedure (G))

2-((5-Methyl-1H-indol-3-yl)methylene)malononitrile

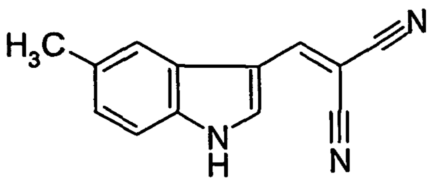

This compound is commercially available from Salor, R94-816-0.

**Example 32**.(General procedure (G))

2-((5-Methyl-1H-indol-3-yl)methylene)malononitrile.

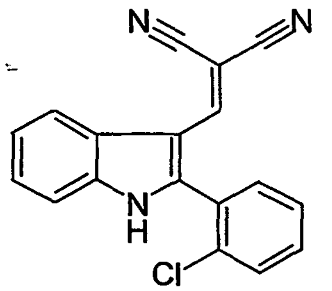

This compound is commercially available from Maybridge, cat. no: RDR01178.

**Example 33** (General procedure (G))

2-(2-Phenyl-3-indolylmethylene)-malononitrile.

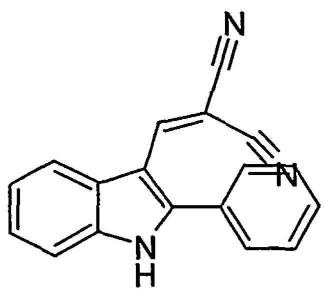

The title compound was prepared from 2-phenyl-3-formylindole and malonitrile in ethanol using a catalytic amount of piperidine.

$^1$H NMR (400 MHz, CHLOROFORM-D ): δ ppm 7.40 (m, 2 H) 7.49 (m, 3 H) 7.60 (m, 3 H) 7.81 (s, 1H) 8.25 (m, 1H) 9.02 (s, 1H); HPLC-MS (Method A): *m/z* = 270 (M+1); $R_t$ = 4.72 min.

**Example 34** (General procedure (G))

2-(2-Chloro-1H-indol-3-ylmethylene)-malononitrile

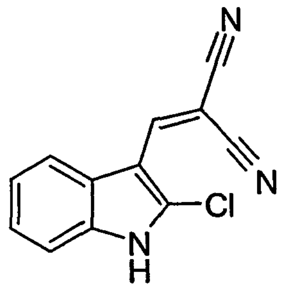

The title compound was prepared from 2-chloro-3-formylindol and malonitrile in ethanol using a catalytic amount of piperidine.

$^1$H NMR (DMSO-$d_6$): δ ppm 7.33 (m, 2 H) 7.48 (d, 1H) 8.07 (d, 1H) 8.19 (s, 1H) 13.75 (s, 1H); HPLC-MS (Method A): *m/z* = 228, 230 (M+1); $R_t$ = 3.41 min.

**Example 35** (General procedure (G))

2-(5-Nitro-1H-indol-3-ylmethylene)-malononitrile.

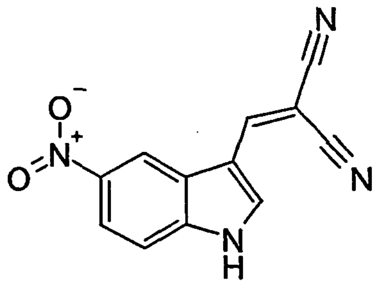

The title compound was prepared from 5-nitro-3-formylindol and malonitrile in ethanol using a catalytic amount of piperidine.

$^1$H NMR (DMSO-$d_6$): δ ppm 7.77 (d, J=8.67 Hz, 1H) 8.17 (dd, *J*=8.67, 2.26 Hz, 1H) 8.72 (s, 1H) 9.01 (s, 1H) 9.14 (d, *J*=2.26 Hz, 1H) 13.02 (m, 1H); HPLC-MS (Method A): *m/z* = 239 (M+1); $R_t$ = 3.51 min.

**Example 36** (General procedure (G))

2-(2-Methyl-5-nitro-1H-indol-3-ylmethylene)-malononitrile.

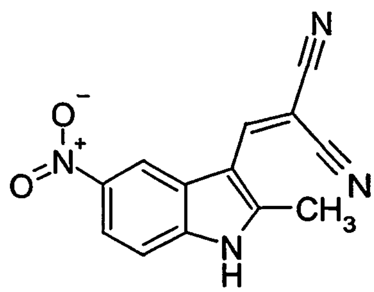

The title compound was prepared from 2-methyl-5-nitro-3-formylindol and malonitrile in ethanol using a catalytic amount of piperidine.

$^1$H NMR (DMSO-$d_6$): δ ppm 7.63 (d, $J$=9.04 Hz, 1H) 8.14 (dd, J=9.04, 2.26 Hz, 1H) 8.48 (s, 1H) 9.05 (d, J=2.26 Hz, 1H) 13.17 (m, 1H); HPLC-MS (Method A): $m/z$ = 253 (M+1); $R_t$ = 3.51 min.

**Example 37** (General procedure (D))

3-Bromo-5-tert-butyl-N-(2-chloro-4-nitro-phenyl)-6-hydroxy-2-methyl-benzamide.

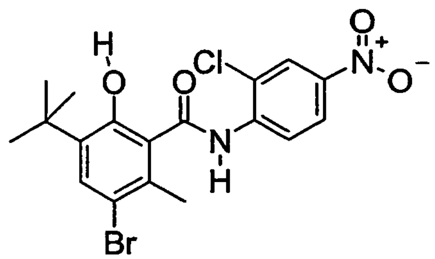

The title compound was prepared from 5-bromo-3-tert-butyl-6-methylsalicylic acid and 2-chlor-4-nitroanilin.

$^1$H-NMR: (CDCl3, 400 MHz): δ ppm 1.41 (m, 9 H) 2.64 (s, 3H) 7.58 (s, 1H) 8.25 (dd, J=9.10, 2.53 Hz, 1H) 8.32 (s, 1H) 8.35 (d, J=2.53 Hz, 1H) 8.85 (d, J=9.10 Hz, 1H) 9.47 (s, 1H); HPLC-MS (Method A): $m/z$ = 443(M+1); $R_t$ = 5.57 min.

**Example 38** (General procedure (D))

N-(2-Chloro-4-nitro-phenyl)-2-hydroxy-3-isopropyl-benzamide.

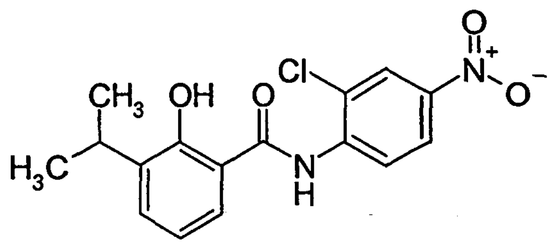

The title compound was prepared from 3-isopropylsalicylic acid and 2-chlor-4-nitroanilin.

$^1$H-NMR: (CDCl3, 400 MHz): δ ppm 1.26 (d, 6 H) 6.93 (t, J=7.36 Hz, 1H) 7.42 (dd, J=18.4 Hz, J=7.36 Hz, 2 H) 8.20 (d, $J$=9.2 Hz, 1H) 8.35 (s, 1H) 8.75 (d, J=9.2 Hz, 1H) 8.82 (s, 1H) 11.77 (s, 1H); HPLC-MS (Method A): $m/z$ = 335, 337(M+1); $R_t$ = 5.18 min.

**Example 39** (General procedure (D))

N-(2-Chloro-4-nitro-phenyl)-2-hydroxy-3-isopropyl-6-methyl-benzamide.

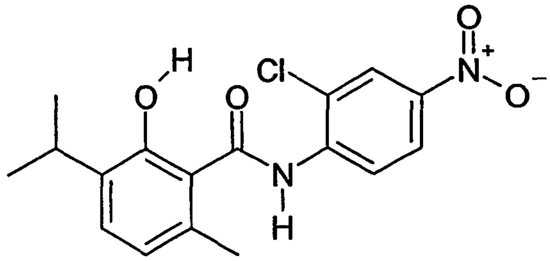

The title compound was prepared from 3-isopropyl-6-methylsalicylic acid and 2-chlor-4-nitroanilin.

$^1$H-NMR: (CDCl3, 400 MHz): δ ppm 1.24 (d, 6 H) 2.68 (s, 3H) 6.79 (d, J=9.2 Hz, 1 H) 7.25 (s, 1H) 8.24 (dd, 1H) 8.36 (s, 1H) 8.54 (s, 1H) 8.85 (d, J=9.2 Hz 1H) 10.25 (s, 1H) ); HPLC-MS (Method A): *m/z* = 349, 351 (M+1); $R_t$ = 4.87 min.

**Example 40** (General procedure (D))

3,5-Di-tert-butyl-N-(2-chloro-4-nitro-phenyl)-2-hydroxy-benzamide.

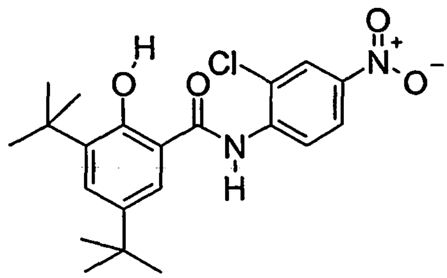

The title compound was prepared from 3,5-di-tertbutylsalicylic acid and 2-chlor-4-nitroanilin.

$^1$H-NMR: (CDCl3, 400 MHz): δ ppm 1.36 (s, 9 H) 1.44 (s, 9 H) 7.39 (s, 1H) 7.58 (s, 1H) 8.23 (m, Hz, 1H) 8.35 (m, 1H) 8.76 (m, 1H) 8.86 (s, 1H) 11.89 (s, 1H).

**Example 41** (General procedure (D))

3-Bromo-N-(2-chloro-4-nitro-phenyl)-6-hydroxy-5-isopropyl-2-methyl-benzamide.

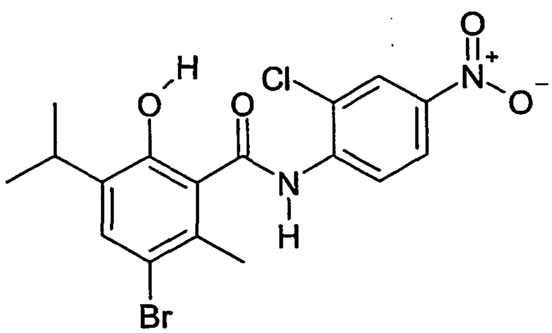

The title compound was prepared from 5-bromo-3-isopropyl-6-methylsalicylic acid and 2-chlor-4-nitroanilin.

$^1$H-NMR: (CDCl3, 400 MHz): δ ppm 1.25 (d, 6 H) 2.67 (m, 3 H) 7.50 (s, 1H) 8.25 (m, 1H) 8.35 (m, 1H) 8.87 (m, 1H).

**Example 42** (General procedure (D))

3-tert-Butyl-5-chloro-N-(4-chloro-3-trifluoromethyl-phenyl)-2-hydroxy-6-methyl-benzamide.

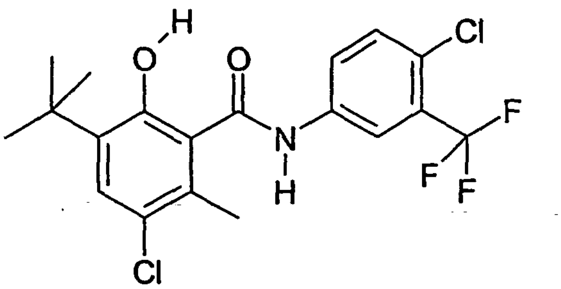

The title compound was prepared from 5-chloro-3-tert-butyl-6-methylsalicylic acid and 3-triflouromethyl-4-chloroanilin.

1H-NMR: (CDCl3, 400 MHz): δ ppm 1.40 (s, 9 H) 2.52 (s, 3 H) 7.39 (s, 1H) 7.60 (m, 1H) 7.66 (m, 1H) 7.73 (s, 1H) 8.37 (d, *J* = 9.2, 1H); HPLC-MS (Method A): *m*/*z* = 420, 422(M+1); $R_t$ = 5.63 min.

**Example 43** (General procedure (D))

3-tert-Butyl-5-chloro-N-(4-cyano-3-trifluoromethyl-phenyl)-2-hydroxy-6-methyl-benzamide.

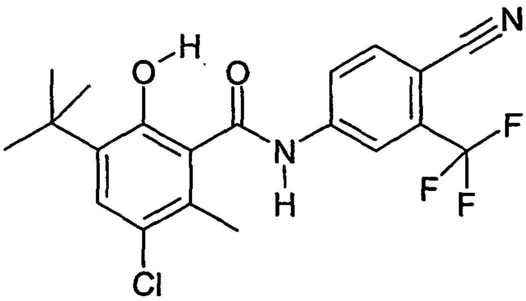

The title compound was prepared from 5-chloro-3-tert-butyl-6-methylsalicylic acid and 4-cyano-3-trifluoromethylanilin.

$^1$H-NMR: (CDCl3, 400 MHz): δ ppm 1.40 (s, 9 H) 2.53 (s, 3 H) 7.39 (s, 1H) 7.66 (s, 1H) 7.86 (d, *J*=8,3 Hz, 1H) 8.97 (d, *J*=8,3 Hz, 1H) 8.05 (s, 1H) 9.05 (s, 1H).

**Example 44** (General procedure (D))

2-Hydroxy-biphenyl-3-carboxylic acid (2-chloro-4-nitro-phenyl)-amide.

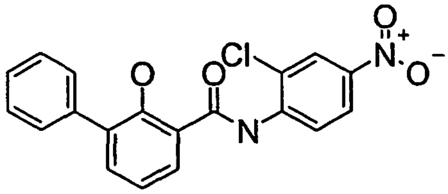

The title compound was prepared from 3-phenylsalicylic acid and 2-chlor-4-nitroanilin.
$^1$H-NMR: (CDCl3, 400 MHz): δ ppm 7.07 (m, 1H) 7.40 (m, 1H) 7.48 (m, 2 H) 7.58 (m, 3 H) 7.69 (m, 1H) 8.22 (m, 1H) 8.35 (s, 1H) 8.79 (m, 1H) 9.13 (m, 1H) 11.20 (s, 1H); HPLC-MS (Method A): *m/z* = 369, 371 (M+1); $R_t$ = 5.05 min.

**Example 45** (General procedure (D))

3-tert-Butyl-N-(2-chloro-4-nitro-phenyl)-2-hydroxy-5-methyl-benzamide.

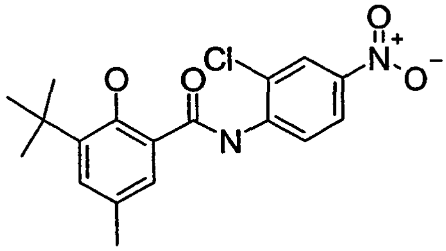

The title compound was prepared from 3-tert-butyl-5-methylsalicylic acid and 2-chlor-4-nitroanilin.
$^1$H-NMR: (CDCl3, 400 MHz): δ ppm 1.42 (s, 9 H) 2.36 (s, 3 H) 7.19 (s, 1H) 7.32 (s, 1H) 8.22 (m, 1H) 8.37 (m, 1H) 8.74 (m, 1H) 8.78 (s, 1H) 11.82 (s, 1H).

**Example 46** (General procedure (D))

N-(2-Chloro-4-nitro-phenyl)-2-hydroxy-6-isopropyl-3-methyl-benzamide.

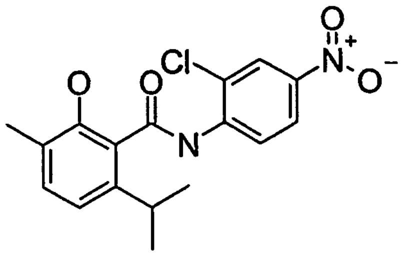

The title compound was prepared from 3-methyl-6-isopropylsalicylic acid and 2-chlor-4-nitroanilin.
$^1$H-NMR: (CDCl3, 400 MHz): δ ppm 1.33 (d, 6 H) 2.25 (s, 3 H) 3.28 (m, 1H) 6.90 (d, J=7.4 Hz, 1H) 7.23 (d, J=7.4 Hz, 1H) 7.82 (s, 1H) 8.22 (m, 2H) 8.33 (s, 1H) 8.85 (m, 1H); HPLC-MS (Method A): *m/z* = 349, 351 (M+1); $R_t$ = 4.85 min.

**Example 47** (General procedure (D))

N-(3,5-Bis-trifluoromethyl-phenyl)-3-tert-butyl-5-chloro-2-hydroxy-6-methyl-benzamide.

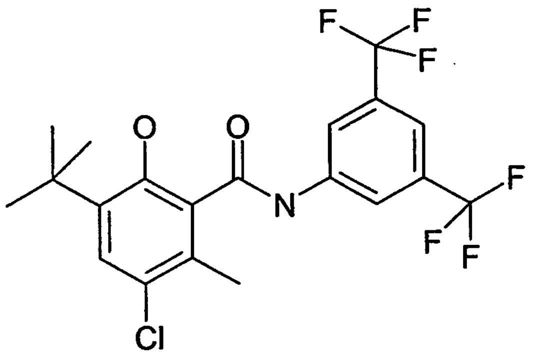

The title compound was prepared from 3-tert-butyl-5-chloro-6-methylsalicylic acid and 3,5-Bis-trifluoromethylanilin.

$^1$H-NMR: (CDCl3, 400 MHz): δ ppm 1.40 (s, 9 H) 2.56 (s, 3 H) 7.39 (s, 1H) 7.69 (s, 1H) 7.70 (m, 1H) 8.10 (m, 2 H); HPLC-MS (Method A): *m/z* = 397(M+1); $R_t$ = 4.75 min.

**Example 48** (General procedure (D))

3-tert-Butyl-5-chloro-N-(2-fluoro-5-trifluoromethyl-phenyl)-2-hydroxy-6-methyl-benzamide.

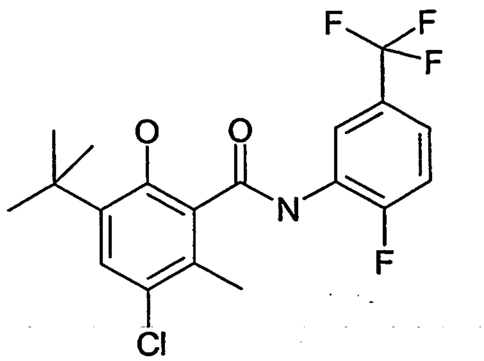

The title compound was prepared from 3-tert-butyl-5-chloro-6-methylsalicylic acid and 2-fluoro-5-trifluoromethylanilin.

$^1$H-NMR: (CDCl3, 400 MHz): δ ppm 1.40 (s, 9 H) 2.57 (s, 3 H) 7.38 (s, 1H) 7.45 (m, 1H) 7.78 (m, 1H) 8.82 (m, 1H).

**Example 49** (General procedure (D))

3-tert-Butyl-5-chloro-2-hydroxy-6-methyl-N-(4-nitro-3-trifluoromethyl-phenyl)-benzamide.

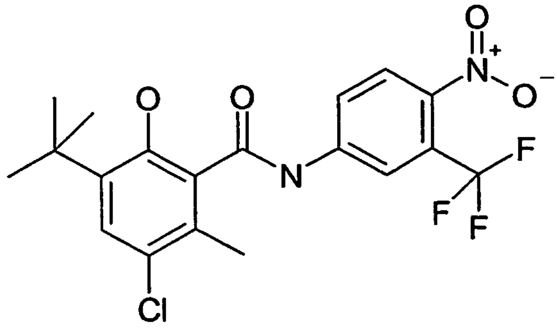

The title compound was prepared from 3-tert-butyl-5-chloro-6-methylsalicylic acid and 4-nitro-3-trifluoromethylanilin.

1H-NMR: (CDCl3, 400 MHz): δ ppm 1.40 (s, 9 H) 2.53 (s, 3 H) 7.39 (s, 1H) 7.70 (m, 1H) 8.04 (m, 2 H) 9.03 (s, 1H). HPLC-MS (Method A): *m*/*z* = 431, 433 (M+1); $R_t$ = 5.4 min.

**Example 50** (General procedure (D))

3-tert-Butyl-5-chloro-2-hydroxy-6-methyl-N-(4-nitro-2-trifluoromethyl-phenyl)-benzamide.

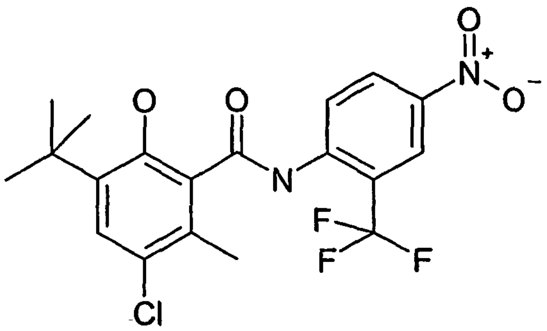

The title compound was prepared from 3-tert-butyl-5-chloro-6-methylsalicylic acid and 4-nitro-2-trifluoromethylanilin.

$^1$H-NMR: (CDCl3, 400 MHz): δ ppm 1.41 (s, 9 H) 2.53 (s, 3 H) 7.42 (s, 1 H) 8.03 (s, 1 H) 8.51 (m, 1 H) 8.59 (m, 1H) 8.89 (m, 1 H) 9.30 (s, 1 H); HPLC-MS (Method A): *m*/*z* = 431, 433 (M+1); $R_t$ = 5.4 min.

**Example 51** (General procedure (D))

3-Bromo-5-tert-butyl-N-(2-chloro-4-cyano-phenyl)-6-hydroxy-2-methyl-benzamide.

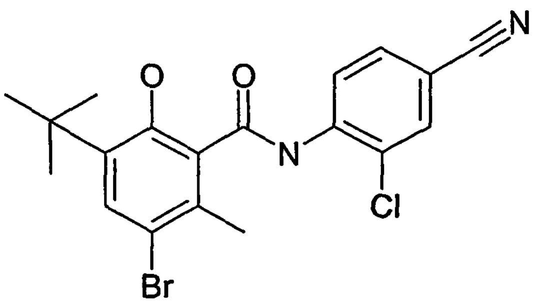

The title compound was prepared from 3-tert-butyl-5-bromo-6-methylsalicylic acid and 2-chloro-4-cyanoanilin.

$^1$H-NMR: (CDCl3, 400 MHz): δ ppm 1.41 (s, 9 H) 2.61 (s, 3 H) 7.57 (s, 1 H) 7.68 (m, 1 H) 7.74 (m, 1 H) 8.23 (s, 1 H) 8.78 (m, 1 H) 9.48 (s, 1H).); HPLC-MS (Method A): *m*/*z* = 430, 432, 434 (M+1); $R_t$ = 5.9 min.

**Example 52** (General procedure (D))

3-Bromo-5-tert-butyl-N-(2-chloro-5-tifluoromethyl-phenyl)-6-hydroxy-2-methyl-benzamide.

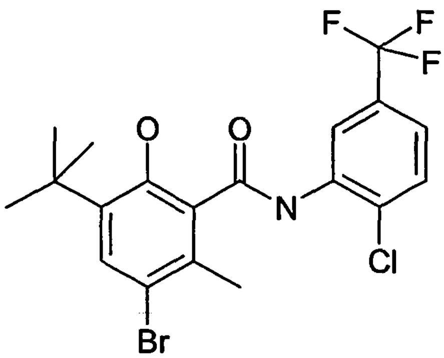

The title compound was prepared from 3-tert-butyl-5-bromo-6-methylsalicylic acid and 2-chloro-5-trifluoromethylanilin.

$^1$H-NMR: (CDCl3, 400 MHz): δ ppm 1.41 (s, 9 H) 2.63 (s, 3 H) 7.4 (m, 1 H) 7.54 (m, 2 H) 8.13 (s, 1 H) 8.90 (s, 1 H) 9.57 (s, 1 H).

**Example 53** (General procedure (D))

3-Bromo-5-tert-butyl-N-(2,4-dichloro-phenyl)-6-hydroxy-2-methyl-benzamide.

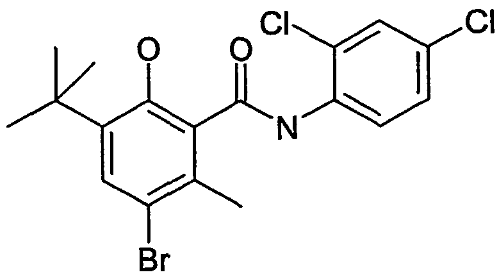

The title compound was prepared from 3-tert-butyl-5-bromo-6-methylsalicylic acid and 2,4-dichloroanilin.

$^1$H-NMR: (CDCl3, 400 MHz): δ ppm 1.4 (s, 9 H) 2.61 (s, 3 H) 7.33 (m, 1 H) 7.46 (m, 1 H) 7.55 (s, 1 H) 8.00 (s, 1 H) 8.50 (m, 1 H) 9.60 (s, 1H).); HPLC-MS (Method A): *m*/*z* = 430, 432, 434 (M+1); $R_t$ = 5.9 min.

**Example 54** (General procedure (D))

3-Bromo-5-tert-butyl-N-(2,4-dichloro-6-nitro-phenyl)-6-hydroxy-2-methyl-benzamide.

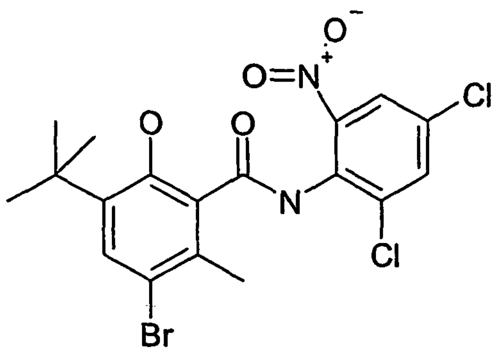

The title compound was prepared from 3-tert-butyl-5-bromo-6-methylsalicylic acid and 2,4-dichloro-6-nitroanilin.

$^1$H-NMR: (CDCl3, 400 MHz): δ ppm 1.40 (s, 9 H) 2.69 (s, 3 H) 7.56 (s, 1 H) 7.78 (m, 1 H) 7.97 (m, 2 H) 9.40 (s, 1H).); HPLC-MS (Method A): *m*/*z* = 497, 499, 501 (M+1); $R_t$ = 5.9 min.

**Example 55** (General procedure (D))

3-Bromo-5-tert-butyl-N-(2,6-dichloro-4-nitro-phenyl)-6-hydroxy-2-methyl-benzamide.

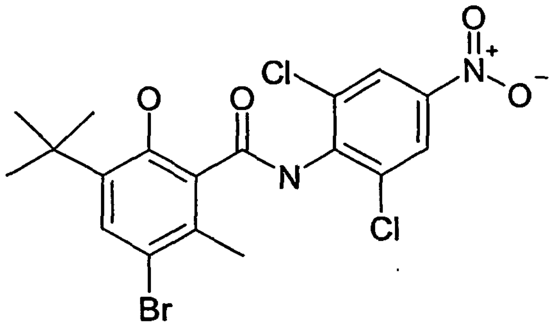

The title compound was prepared from 3-tert-butyl-5-bromo-6-methylsalicylic acid and 2,6-dichloro-4-nitroanilin.

$^1$H-NMR: (CDCl3, 400 MHz): δ ppm 1.41 (s, 9 H) 2.71 (s, 3 H) 7.35 (s, 1 H) 7.58 (s, 1 H) 8.32 (m, 2 H) 9.40 (s, 1H).); HPLC-MS (Method A): *m*/*z* = 475, 477, 479 (M+1); $R_t$ = 5.6 min.

**Example 56** (General procedure (D))

3-Bromo-5-tert-butyl-N-{5-chloro-4-[(4-chloro-phenyl)-cyano-methyl]-2-methyl-phenyl}-6-hydroxy-2-methyl-benzamide.

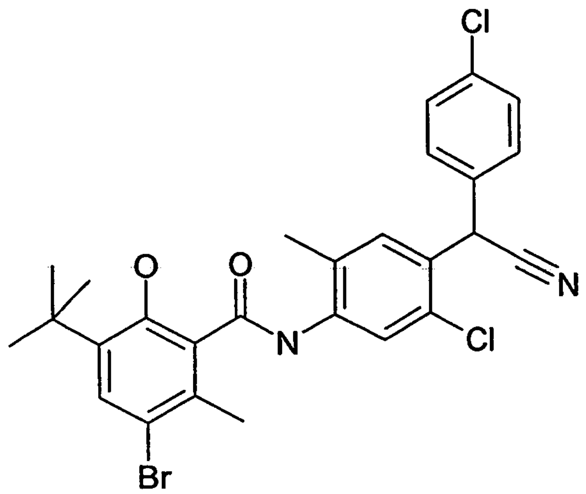

The title compound was prepared from 3-tert-butyl-5-bromo-6-methylsalicylic acid and 5-chloro-4-[(4-chloro-phenyl)-cyano-methyl]-2-methylanilin.
$^1$H-NMR: (CDCl3, 400 MHz): δ ppm 1.41 (s, 9 H) 2.31 (s, 3 H) 2.60 (s, 3 H) 7.33 (m, 6 H) 7.54 (s, 1 H) 8.29 (s, 1 H) 9.40 (s, 1 H); HPLC-MS (Method A): *m/z* = 559, 561, 563 (M+1); $R_t$ = 5.92 min.

**Example 57** (General procedure (D))

3-Bromo-6-hydroxy-5-isopropyl-2-methyl-N-(4-nitro-2-trifluoromethyl-phenyl)-benzamide.

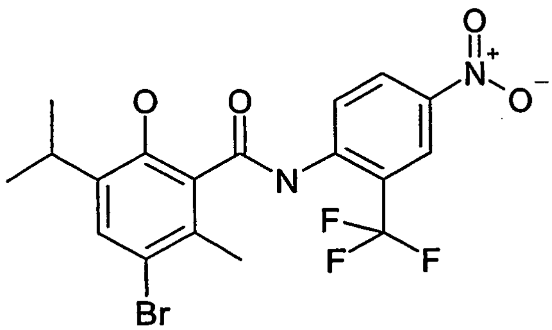

The title compound was prepared from 3-isopropyl-5-bromo-6-methylsalicylic acid and 4-nitro-2-trifluoromethylanilin.
$^1$H-NMR: (CDCl3, 400 MHz): δ ppm 1.25 (d, 6H) 2.56 (s, 3H,) 7.52 (s, 1 H) 8.04 (s, 1 H) 8.51 (m, 1 H) 8.59 (m, 1 H) 8.86 (m, 1H); HPLC-MS (Method A): *m/z* = 461, 463 (M+1); $R_t$ = 4.9 min.

**Example 58** (General procedure (D))

3-Bromo-5-tert-butyl-6-hydroxy-2-methyl-N-(4-nitro-2-trifluoromethyl-phenyl)-benzamide.

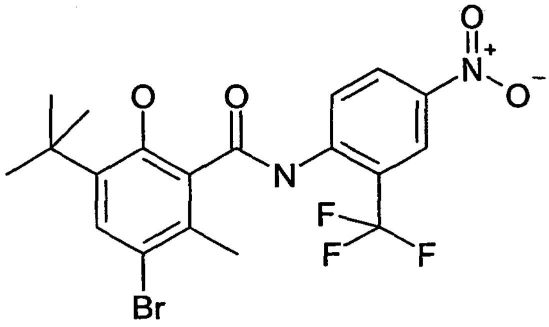

The title compound was prepared from 3-tert-butyl-5-bromo-6-methylsalicylic acid and 4-nitro-2-trifluoromethylanilin.

$^1$H-NMR: (CDCl3, 400 MHz): δ ppm 1.41 (s, 9 H) 2.53 (s, 3 H) 7.58 (s, 1 H) 8.02 (s, 1 H) 8.52 (m, 1 H) 8.59 (m, 1 H) 8.89 (m, 1 H) 9.20 (s, 1H); HPLC-MS (Method A): *m/z* = 475, 477(M+1); $R_t$ = 5.5 min.

**Example 59** (General procedure (D))

3-tert-Butyl-2-hydroxy-6-methyl-N-(4-nitro-2-trifluoromethyl-phenyl)-benzamide.

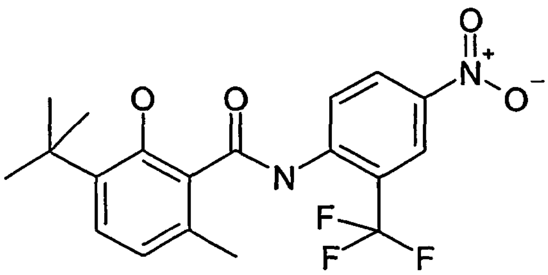

The title compound was prepared from 3-tert-butyl-6-methylsalicylic acid and 4-nitro-2-trifluoromethylanilin.

$^1$H-NMR: (CDCl3, 400 MHz): δ ppm 1.43 (s, 9 H) 2.57 (s, 3 H) 6.75 (d, *J*=7.36 Hz, 1 H) 7.34 (d, *J*=7.36 Hz, 1 H) 8.18 (s, 1 H) 8.51 (m, 1 H) 8.59 (m, 1 H) 8.83 (m, 1 H) 10.24 (s, 1 H).

**Example 60** (General procedure (D))

3-Bromo-N-(2-bromo-3,5-bis-trifluoromethyl-phenyl)-5-tert-butyl-6-hydroxy-2-methyl-benzamide.

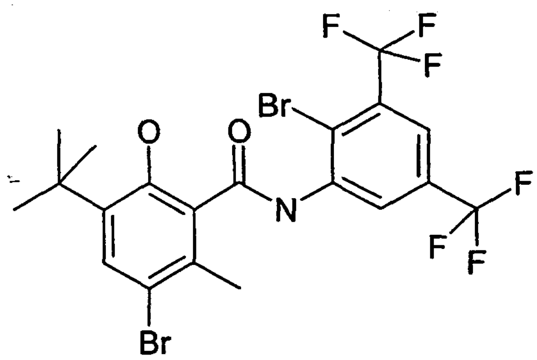

The title compound was prepared from 3-tert-butyl-5-bromo-6-methylsalicylic acid and 2-bromo-3,5-bis-trifluoromethylanilin.

$^1$H-NMR: (CDCl3, 400 MHz): δ ppm 1.41 (s, 9 H) 2.65 (s, 3 H) 7.56 (s, 1 H) 7.35 (m, 1 H) 8.40 (s, 1 H) 9.13 (s, 1 H) 9.35 (s, 1 H); HPLC-MS (Method A): *m/z* = 576, 578, 580 (M+1); $R_t$ = 6.1 min.

**Example 61** (General procedure (D))

N-(2,5-Bis-trifluoromethyl-phenyl)-3-bromo-5-tert-butyl-6-hydroxy-2-methyl-benzamide.

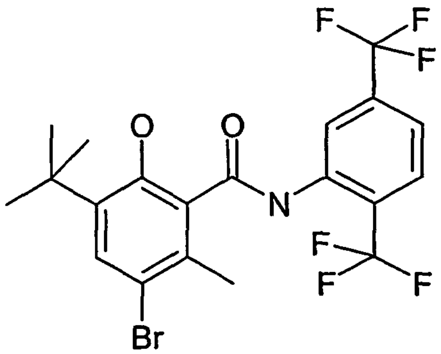

The title compound was prepared from 3-tert-butyl-5-bromo-6-methylsalicylic acid and 2,5-bis-trifluoromethylanilin.

$^1$H-NMR: (CDCl3, 400 MHz): δ ppm 1.40 (s, 9 H) 2.56 (s, 3 H) 7.57 (m, 2 H) 7.83 (m, 2 H) 8.82 (s, 1 H) 9.35 (s, 1 H); HPLC-MS (Method A): *m/z* = 430, 432, 434 (M+1); $R_t$ = 5.9 min. ); HPLC-MS (Method A): *m/z* = 498, 500 (M+1); $R_t$ = 5.8 min.

**Example 62** (General procedure (D))

3-Bromo-5-tert-butyl-N-(2,4-dichloro-6-tifluoromethyl-phenyl)-6-hydroxy-2-methyl-benzamide.

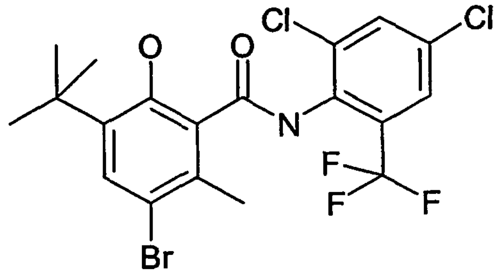

The title compound was prepared from 3-tert-butyl-5-bromo-6-methylsalicylic acid and 2,4-dichloro-6-trifluoromethylanilin.

$^1$H-NMR: (CDCl3, 400 MHz): δ ppm 1.40 (s, 9 H) 2.69 (s, 3 H) 7.15 (s, 1 H) 7.56 (s, 1 H) 7.68 (s, 1 H) 7.76 (s, 1 H) 9.50 (s, 1 H). ); HPLC-MS (Method A): *m/z* = 498, 500, 502 (M+1); $R_t$ = 5.9 min.

**Example 63** (General procedure (D))

3-Bromo-5-tert-butyl-6-hydroxy-N-(4-isopropyl-2-trifluoromethyl-phenyl)-2-methyl-benzamide.

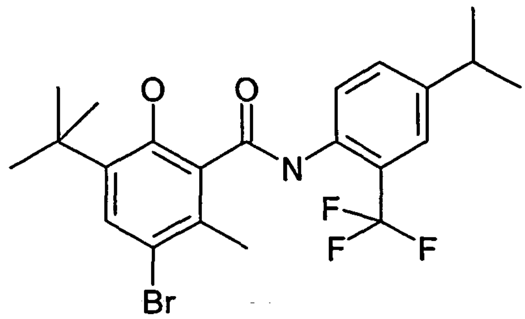

The title compound was prepared from 3-tert-butyl-5-bromo-6-methylsalicylic acid and 4-isopropyl-6-trifluoromethylanilin.

$^1$H-NMR: (CDCl3, 400 MHz): δ ppm 1.27 (d, 6 H) 1.41 (s, 9 H) 7.50 (m, 3 H) 7.68 (s, 1 H) 8.20 (m, 1 H) 9.50 (s, 1 H).

**Example 64** (General procedure (D))

N-(3,5-Bis-trifluoromethyl-phenyl)-3-fluoro-5-trifluoromethyl-benzamide.

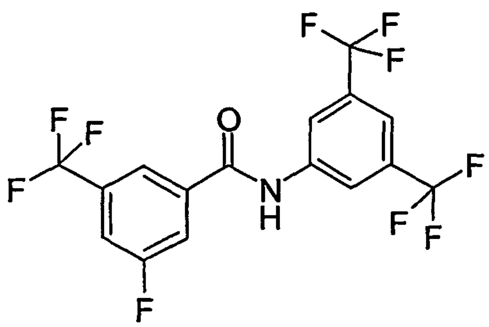

The title compound was prepared from 3-fluoro-5-trifluoromethyl-benzoic acid and 3,5-Bis-trifluoromethylanilin.
$^1$H NMR (DMSO-$d_6$): δ ppm 7.88 (m, 1 H) 8.04 (d, 1 H) 8.15 (d, 1 H) 8.22 (m, 1 H) 8.48 (m, 2 H) 11.04 (s, 1 H); HPLC-MS (Method A): *m*/*z* = 420 (M+1); $R_t$ = 5.5 min.

**Example 65** (General procedure (D))

3-Fluoro-N-(4-nitro-3-trifluoromethyl-phenyl)-5-trifluoromethyl-benzamide.

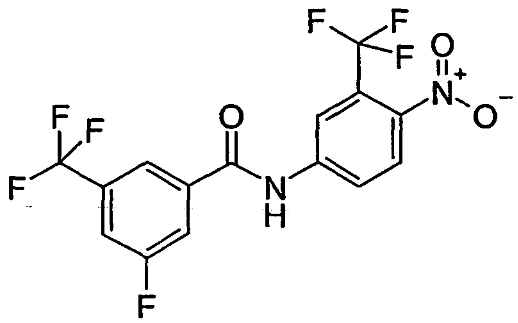

The title compound was prepared from 3-fluoro-5-trifluoromethyl-benzoic acid and 4-nitro-3-trifluoromethylanilin.
$^1$H NMR (DMSO-$d_6$): δ ppm 8.05 (d, 1 H) 8.16 (d, 1 H) 8.21 (s, 1 H) 8.32 (m, 2 H) 8.43 (d, *J*=1.88 Hz, 1 H) 11.18 (s, 1 H); HPLC-MS (Method A): *m*/*z* = 397 (M+1); $R_t$ = 5.0 min.

**Example 66** (General procedure (D))

N-(3,5-Bis-trifluoromethyl-phenyl)-3-fluoro-4-trifluoromethyl-benzamide.

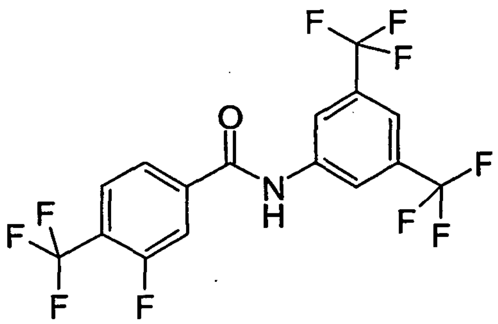

The title compound was prepared from 3-fluoro-4-trifluoromethylbenzoic acid and 3,5-Sis-trifluoromethylanilin.
$^1$H NMR (DMSO-$d_6$): δ ppm 7.77 (m, 1 H) 7.87 (m, 1 H) 8.29 (m, 2 H) 8.49 (m, 2 H) 11.02 (s, 1 H); HPLC-MS (Method A): *m*/*z* = 420 (M+1); $R_t$ = 5.4 min.

**Example 67** (General procedure (D))

4-Fluoro-N-(4-nitro-3-trifluoromethyl-phenyl)-3-trifluoromethyl-benzamide.

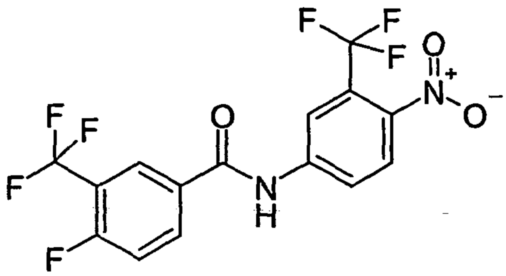

The title compound was prepared from 4-fluoro-3-trifluoromethylbenzoic acid and 4-nitro-3-trifluoromethylanilin.

$^1H$ NMR (DMSO-$d_6$): δ ppm 7.77 (m, 1 H) 8.29 (s, 1 H) 8.31 (d, *J*=2.26 Hz, 1 H) 8.39 (m, 3 H) 11.15 (m, 1 H); HPLC-MS (Method A): *m*/*z* = 397 (M+1); $R_t$ = 4.9 min.

**Example 68** (General procedure (F))

3-(3,5-Di-tert-butyl-4-hydroxy-phenyl)-2-(2,2-dimethyl-propionyl)-acrylonitrile

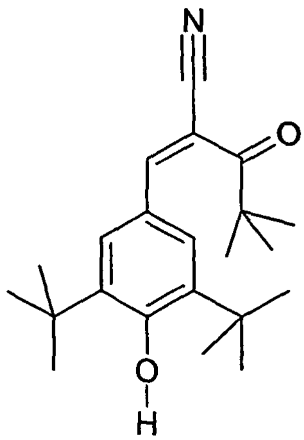

The title compound was prepared from 2-phenyl-3-formylindole and malonitrile in ethanol using a catalytic amount of piperidine.

$^1H$-NMR: (CDCl3, 300 MHz): δ ppm 1.42 (s, 9 H) 1.47 (s, 18 H) 5.90 (s, 1 H) 7.94 (s, 2 H) 8.20 (s, 1 H); HPLC-MS (Method A): *m*/*z* = 342 (M+1); $R_t$ = 6.0 min.

**Example 69** (General procedure (F))

2-Acetyl-3-(3,5-di-tert-butyl-4-hydroxy-phenyl)-acrylic acid ethyl ester.

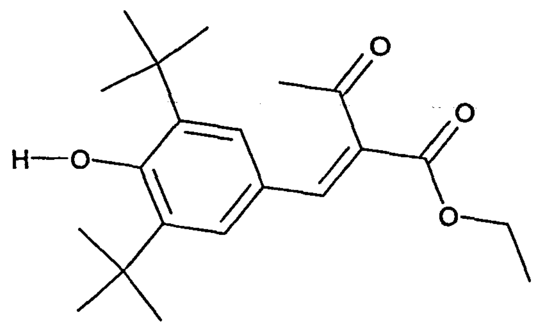

This compound is commercially available from Sigma, cat. no; S482102.

**Example 70** (General procedure (F))

2-(3,5-Dimethyl-4-hydroxy-benzylidene)-malononitrile

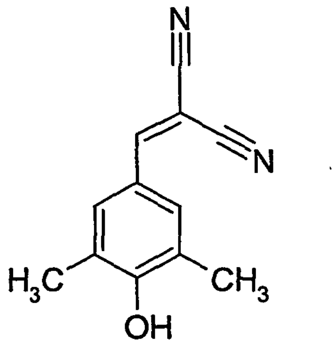

The title compound was prepared from 3,5-dimethyl-4-hydroxybenzaldehyde and cyanoacetonitrile in ethanol using a catalytic amount of piperidine

$^{1}$H-NMR: (CDCl3, 300 MHz): δ ppm 2.30 (s, 6 H) 7.57 (s, 1 H) 7.61 (s, 2 H); HPLC-MS (Method A): *m*/*z* = 199 (M+1); $R_t$ = 3.51 min.

**Example 71** (General procedure (A))

2-(3,5-Dimethyl-4-hydroxy-benrylidene)-malononitrile

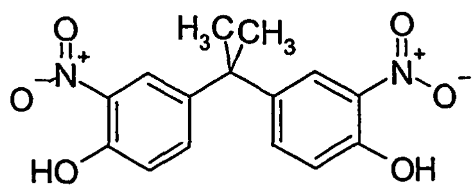

This compound is commercially available from Salor cat. No. S13,586-0.

**Example 72** (General procedure (A))

2,6-Di-tert-butyl-4-nitro-phenol.

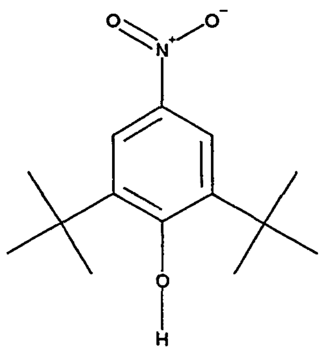

This compound is commercially available from Bionet, cat.no: 7L-850.

**Example 73** (General procedure (A))

2-tert-Butyl-4,6-dinitro-phenol.

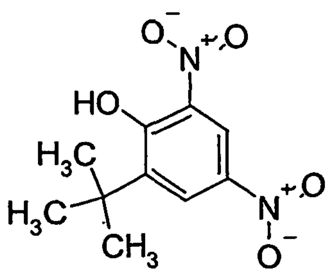

This compound is commercially available from Riedel-de-Haën cat. No. 36775.

**Example 74** (General procedure (F))

3-(3,5-Di-tert-butyl-4-hydroxy-phenyl)-2-pyridin-2-yl-acrylonitrile.

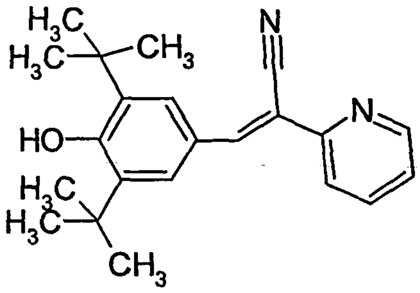

The title compound was prepared from 3,5-di-tert-butyl-4-hydroxybenzaldehyde and 2-pyridylacetonitrile in 35 % yield.

$^1$H NMR (DMSO-$d_6$): δ ppm 1.43 (s, 18 H) 7.39 (dd, *J*=6.41, 4.90 Hz, 1 H) 7.82 (m, *J*=8.67, 8.67 Hz, 2 H) 7.92 (s, 2 H) 8.39 (s, 1 H) 8.64 (d, *J*=4.14 Hz, 1 H); HPLC-MS (Method A): *m*/*z* = 335 (M+1); $R_t$ = 5.48 min..

**Example 75** (General procedure (F))

2-[1-(3,5-Di-tert-butyl-4-hydroxy-phenyl)-ethylidene]-malononitrile.

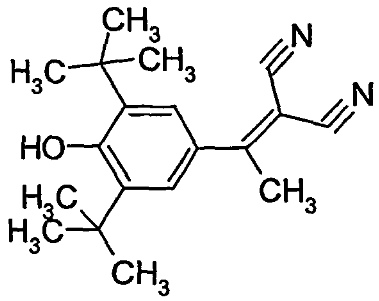

The title compound was prepared from 3,5-di-tert-butyl-4-hydroxyacetophenone and malononitrile in 10 % yield.

$^1$H-NMR: (CDCl3, 300 MHz): δ ppm 1.45 (s, 18 H) 2.62 (s, 3 H) 5.79 (s, 1 H) 7.50 (s, 2 H); HPLC-MS (Method A): *m*/*z* = 297 (M+1); $R_t$ = 5.12 min.

**Example 76** (General procedure (F))

3-(3,5-Di-tertbutyl-4-hydroxybenzylidene)-2-(diethylphosphonate)-propenenitrile.

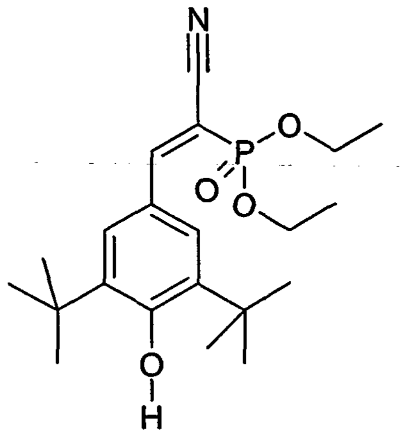

The title compound was prepared from 3,5-di-tert-butyl-4-hydroxybenzaldehyde and diethyl cyanomethylphosphonate in 7 % yield.

$^1$H-NMR: (CDCl3, 300 MHz): δ ppm 1.40 (t, *J*=7.07 Hz, 6 H) 1.47 (s, 18 H) 4.20 (m, 4 H) 5.88 (s, 1 H) 7.90 (s, 2 H) 7.96 (s, 1 H); HPLC-MS (Method A): *m*/*z* = 394 (M+1); $R_t$ = 3.8 min.

**Example 77** (General procedure (F))

3-(3,5-Di-tert-butyl-4-hydroxy-phenyl)-2-(4-nitro-phenyl)-acrylonitrile.

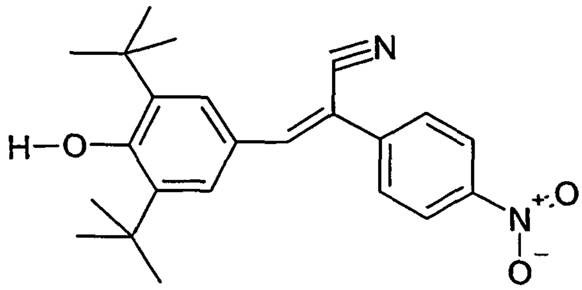

The title compound was prepared from 3,5-di-tert-butyl-4-hydroxybenzaldehyde and 4-nitrophenylacetonitrile in 62 % yield.

$^1$H NMR (DMSO-$d_6$): δ ppm 1.43 (s, 18 H) 7.91 (s, 1 H) 7.95 (s, 2 H) 8.00 (d, *J*=8.67 Hz, 2 H) 8.24 (s, 1 H) 8.33 (d, *J*=8.67 Hz, 2 H); HPLC-MS (Method A): *m*/*z* = 379 (M+1); $R_t$ = 5.7 min.

**Example 78** (General procedure (F))

3-(3,5-Di-tert-butyl-4-hydroxy-phenyl)-2-pyridin-4-yl-acrylonitrile.

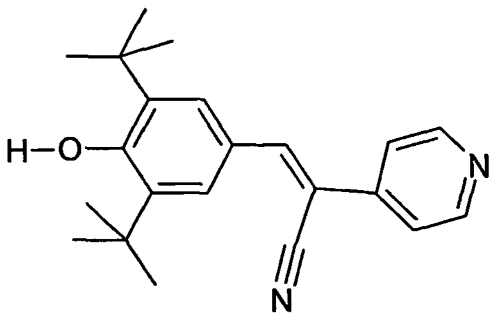

The title compound was prepared from 3,5-di-tert-butyl-4-hydroxybenzaldehyde and 2-pyridylacetonitrile in 75 % yield.

$^1H$ NMR (DMSO-$d_6$): δ ppm 1.43 (s, 18 H) 7.71 (d, $J$=6.03 Hz, 2 H) 8.27 (s, 1 H) 8.65 (d, $J$=6.03 Hz, 2 H); HPLC-MS (Method A): $m/z$ = 335 (M+1); $R_t$ = 3.7 min.

**Example 79** (General procedure (F))

2-(3,5-Bis-trifluoromethyl-phenyl)-3-(3,5-di-tert-butyl-4-hydroxy-phenyl)-acrylonitrile.

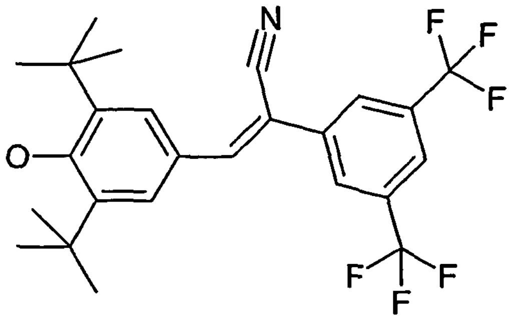

The title compound was prepared from 3,5-di-tert-butyl-4-hydroxybenzaldehyde and 3,5-bis-trifluoromethyl-phenylacetonitrile in 20 % yield

$^1H$ NMR (DMSO-$d_6$): ppm 1.51 (m, 18 H) 5.78 (s, 1 H) 7.57 (s, 1 H) 7.85 (s, 1 H) 7.86 (s, 2 H) 8.07 (s, 1 H).

**Example 80** (General procedure (F))

3-(3,5-Di-tert-butyl-4-hydroxy-phenyl)-2-(4-trifluoromethoxy-phenyl)-acrylonitrile.

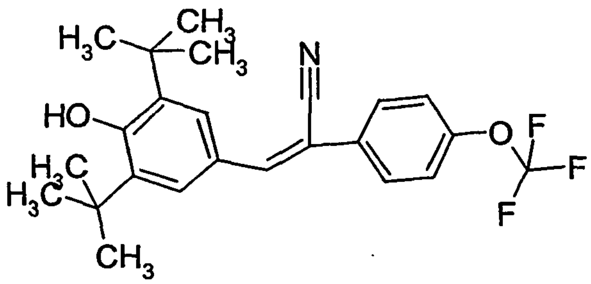

The title compound was prepared from 3,5-di-tert-butyl-4-hydroxybenzaldehyde and 4-trifluoromethoxy-phenylacetonitrile in 10 % yield

$^1$H NMR (DMSO-$d_6$): δ ppm 1.49 (s, 18 H) 5.68 (s, 1 H) 7.27 (d, 2 H) 7.45 (s, 1 H) 7.68 (d, *J*=9.04 Hz, 2 H) 7.81 (s, 2 H); HPLC-MS (Method A): *m*/*z* = 418 (M+1); $R_t$ = 6.2 min.

**Example 81** (General procedure (F))

3-(3,5-Di-tert-butyl-4-hydroxy-phenyl)-2-(4-trifluoromethyl-phenyl)-acrylonitrile.

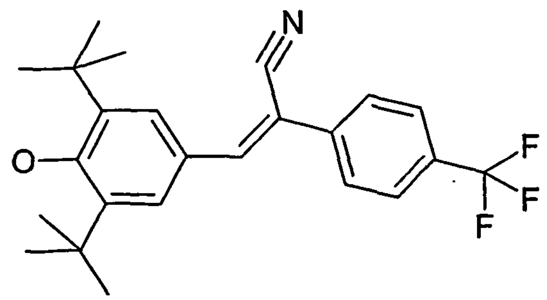

The title compound was prepared from 3,5-di-tert-butyl-4-hydroxybenzaldehyde and 4-trifluoromethyl-phenylacetonitrile in 31 % yield

$^1$H NMR (DMSO-$d_6$): δ ppm 1.43 (s, 18 H) 7.85 (d, *J*=8.29 Hz, 2 H) 7.92 (s, 2 H) 7.94 (d, 2 H) 8.15 (s, 1 H).

**Example 82** (General procedure (F))

2-Cyano-3-(3,5-di-tert-butyl-4-hydroxy-phenyl)-but-2-enoic acid ethyl ester.

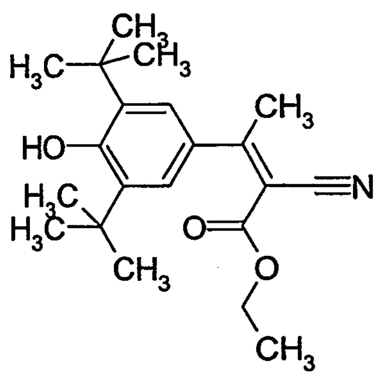

The title compound was prepared from 3,5-di-tert-butyl-4-hydroxyacetophenone and ethy cyanoacetic acid in 19 % yield.

$^1$H NMR (DMSO-$d_6$): δ ppm 1.27 (t, 3 H) 1.39 (s, 18 H) 2.62 (s, 3 H) 4.24 (q, 2 H) 7.37 (s, 2 H) 7.62 (s, 1 H); HPLC-MS (Method A): *m*/*z* = 344 (M+1); $R_t$ = 5.4 min.

**Example 83** (General procedure (F))

N-(4-Chloro-phenyl)-2-cyano-3-(3,5-di-tert-butyl-4-hydroxy-phenyl)-acrylamide.

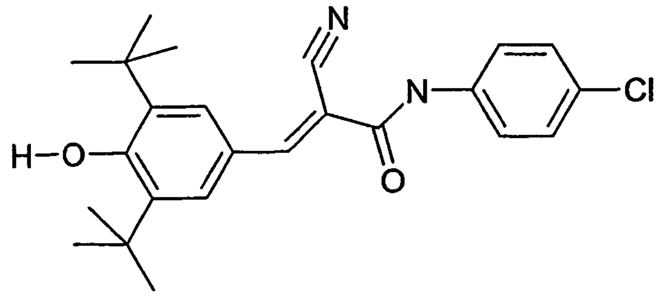

The title compound was prepared from 3,5-di-tert-butyl-4-hydroxybenzaldehyde and 4'-chloro-2-cyanoacetanilide in 53 % yield

$^1H$ NMR (DMSO-$d_6$): δ ppm 1.42 (s, 18 H) 7.42 (d, $J$=9.04 Hz, 2 H) 7.70 (d, $J$=8.67 Hz, 2 H) 7.91 (s, 2 H) 8.09 (s, 1 H) 8.21 (s, 1 H) 10.32 (s, 1 H); HPLC-MS (Method A): $m/z$ = 412 (M+1); $R_t$ = 5.1 min.

**Example 84** (General procedure (F))

(E)-3-(3,5-Di-tert-butyl-4-hydroxy-phenyl)-2-methanesulfonyl-acrylonitrile

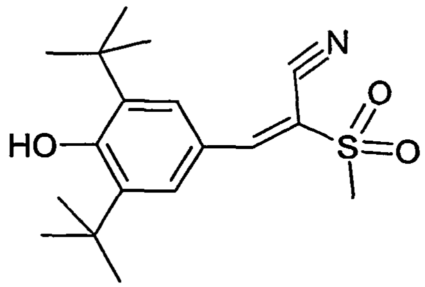

Step A: The title compound was prepared from 3,5-di-tert-butyl-4-hydroxybenzaldehyde and methanesulfonylacetonitrile in 35 % yield.

$^1H$ NMR ($CDCl_3$): δ 1.47 (s, 18 H) 3.18 (s, 3 H) 6.03 (s, 1 H) 7.86 (s, 2 H) 8.04 (s, 1 H); HPLC-MS (Method A): $m/z$ = 358 (M+Na); $R_t$ = 4.79 min.

**Example 85** (General procedure (F))

(E)-2-(4-Chloro-benzenesulfonyl)-3-(3,5-di-tert-butyl-4-hydroxy-phenyl)-acrylonitrile

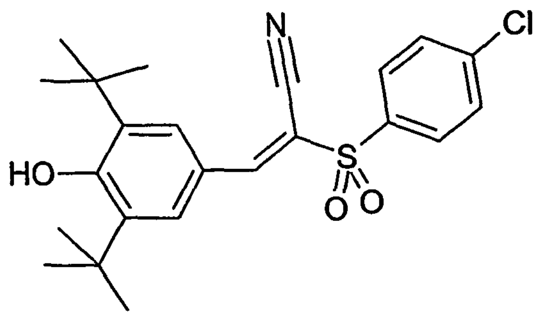

Step A: The title compound was prepared from 3,5-di-tert-butyl-4-hydroxybenzaldehyde and 4-chlorophenylsulfonylacetonitrile in 72 % yield

$^1$H NMR ($CDCl_3$): δ 1.45 (s, 18 H) 6.01 (s, 1 H) 7.56 (d, *J*=8.59 Hz, 2 H) 7.83 (s, 2 H) 7.94 (d, *J*=8.59 Hz, 2 H) 8.12 (s, 1 H); HPLC-MS (Method A): *m*/*z* = 432 (M+1); $R_t$ = 4.88 min.

**Example 86** (General procedure (F))

(E)-3-(3,5-Di-tert-butyl-4-hydroxy-phenyl)-2-(4-fluoro-benzenesulfonyl)-acrylonitrile

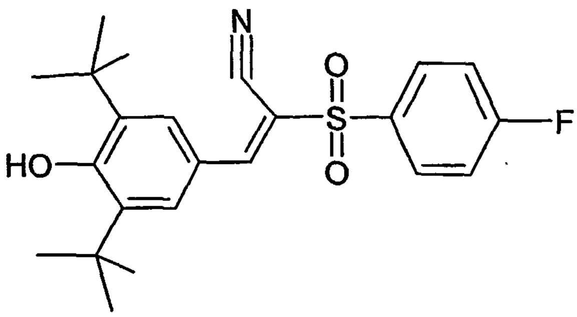

Step A: The title compound was prepared from 3,5-di-tert-butyl-4-hydroxybenzaldehyde and 4-fluoronitrophenylsulfonylacetonitrile in 37 % yield

$^1$H NMR (DMSO-*d*$_6$): δ ppm 1.39 (m, 18 H) 7.58 (dd, *J*=8.84 Hz, 2 H) 7.95 (s, 2 H) 8.08 (dd, *J*=9.10, 5.05 Hz, 2 H) 8.44 (s, 1 H) 8.46 (s, 1 H).

Example 87 (General procedure (F))

(E)-2-Benzenesulfonyl-3-(3,5-di-tert-butyl-4-hydroxy-phenyl)-acrylonitrile

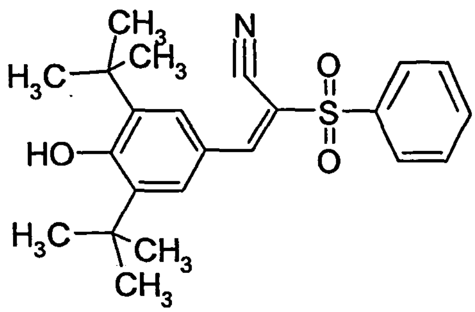

Step A: The title compound was prepared from 3,5-di-tert-butyl-4-hydroxybenzaldehyde and phenylsulfonylacetonitrile in 61 % yield

$^1$H NMR (DMSO-*d*$_6$): δ ppm 1.39 (s, 18 H) 7.76 (m, 3 H) 7.95 (s, 2 H) 8.00 (d, *J*=7.16 Hz, 2 H) 8.44 (s, 2 H); HPLC-MS (Method #): *m*/*z* = 399 (M+1); $R_t$ = 5.5 min.

**Example 88** (General procedure (F))

(E)-3-(3,5-Di-tert-butyl-4-hydroxy-phenyl)-2-(propane-2-sulfonyl)-acrylonitrile

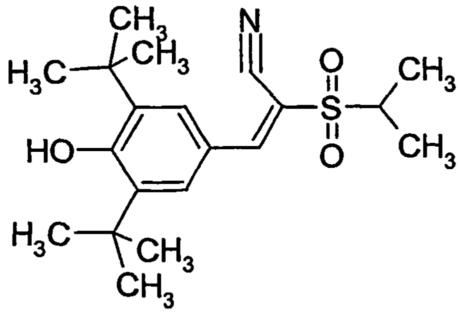

Step A: The title compound was prepared from 3,5-di-tert-butyl-4-hydroxybenzaldehyde and propane-2-sulphonylacetonitrile in 34 % yield

$^1$H NMR (DMSO-$d_6$): δ ppm 1.32 (d, *J*=6.78 Hz, 6 H) 1.41 (s, 18 H) 3.56 (m, 1 H) 7.98 (s, 2 H) 8.16 (s, 1 H) 8.41 (s, 1 H); HPLC-MS (Method A): *m*/*z* = 464 (M+1); $R_t$ = 5.0 min.

**Example 89** (General procedure (F))

3-(3,5-Di-tert-butyl-4-hydroxy-phenyl)-2-(2,5-dichloro-benzenesulfonyl)-acrylonitrile

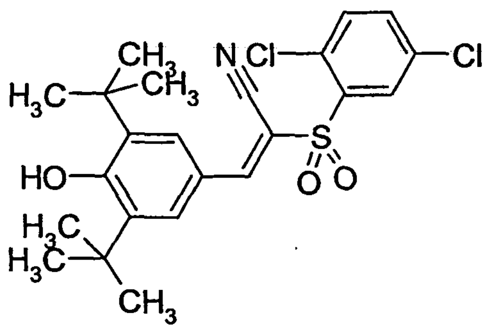

Step A: The title compound was prepared from 3,5-di-tert-butyl-4-hydroxybenzaldehyde and 2,5-dichlorophenylsulfonylacetonitrile in 68 % yield.

$^1$H NMR (DMSO-$d_6$): δ ppm 1.39 (s, 18 H) 7.82 (d, *J*=8.29 Hz, 1 H) 7.93 (dd, 1 H) 8.00 (s, 2 H) 8.16 (d, *J*=2.26 Hz, 1 H) 8.50 (s, 1 H) 8.58 (m, 1 H); HPLC-MS (Method A): *m*/*z* = 465, 467 (M+1); $R_t$ = 6.0 min.

**Example 90** (General procedure (F))

3-(3,5-Di-tert-butyl-4-hydroxy-phenyl)-2-(2,4-dichloro-benzenesulfonyl)-acrylonitrile

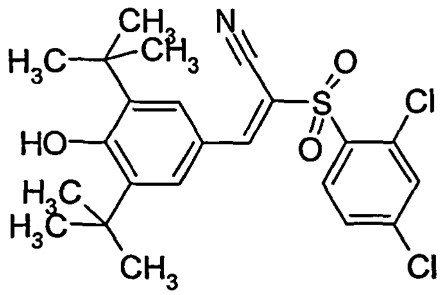

Step A: The title compound was prepared from 3,5-di-tert-butyl-4-hydroxybenzaldehyde and 3,5-dichlorophenylsulfonylacetonitrile in 68 % yield.

$^1$H NMR (DMSO-$d_6$): δ ppm 1.39 (s, 18 H) 7.82 (dd, $J$=8.48, 2.07 Hz, 1 H) 8.01 (m, 3 H) 8.21 (d, $J$=8.67 Hz, 1 H) 8.48 (s, 1 H) 8.55 (s, 1 H); HPLC-MS (Method A): $m/z$ = 467 (M+1); $R_t$ = 6.1 min.

**Example 91** (General procedure (F))

3-(3,5-Di-tert-butyl-4-hydroxy-phenyl)-2-(hexane-1-sulfonyl)-acrylonitrile.

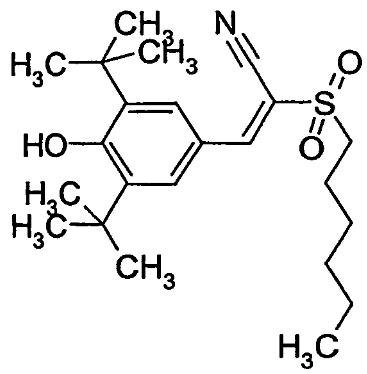

Step A: The title compound was prepared from 3,5-di-tert-butyl-4-hydroxybenzaldehyde and hexane-1-sulfonylacetonitrile in 68 % yield

$^1$H NMR (DMSO-$d_6$): δ 1 H NMR (400 MHz, DMSO-D6) □ ppm 0.85 (m, 3 H) 1.26 (m. 4 H) 1.40 (m, 2 H) 1.40 (s, 18 H) 1.69 (m, 2 H) 3.40 (m, 2 H) 7.95 (s, 2 H) 8.17 (s, 1 H) 8.40 (s, 1 H); HPLC-MS (Method A): $m/z$ = 407 (M+1); $R_t$ = 6.4 min.

**Example 92** (General procedure (F))

2-(4-Bromo-benzenesulfonyl)-3-(3,5-di-tert-butyl-4-hydroxy-phenyl)-acrylonitrile

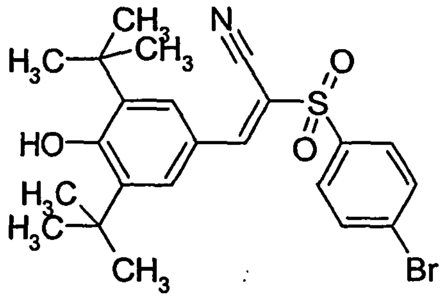

Step A: The title compound was prepared from 3,5-di-tert-butyl-4-hydroxybenzaldehyde and 4-bromophenylsulfonylacetonitrile in 53 % yield.

$^1$H NMR (DMSO-$d_6$): δ ppm 1.38 (s, 18 H) 7.93 (d, *J*=4.52 Hz, 6 H) 8.41 (s, 1 H) 8.46 (m, 1 H); HPLC-MS (Method A): *m*/*z* = 475, 477 (M+1); $R_t$ = 5.7 min.

**Example 93** (General procedure (F))

2-(3-Bromo-benzenesulfonyl)-3-(3,5-di-tert-butyl-4-hydroxy-phenyl)-acrylonitrile

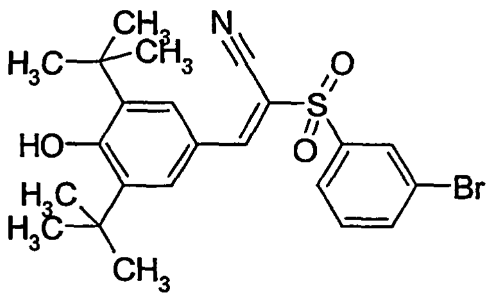

Step A: The title compound was prepared from 3,5-di-tert-butyl-4-hydroxybenzaldehyde and 3-bromophenylsulfonylacetonitrile in 53 % yield.

$^1$H NMR (DMSO-$d_6$): δ ppm 1.39 (s, 18 H) 7.69 (t, *J*=7.91 Hz, 1 H) 7.96 (s, 2 H) 8.02 (t, *J*=6.97 Hz, 2 H) 8.15 (d, *J*=1.88 Hz, 1 H) 8.47 (s, 1 H) 8.53 (s, 1 H); HPLGMS (Method A): *m*/*z* = 475, 477 (M+1); $R_t$ = 5.7 min.

**Example 94** (General procedure (D))

5-Bromo-3-tert-butyl-N-(2-chloro-4-cyanophenyl)-2-hydroxybenzamide.

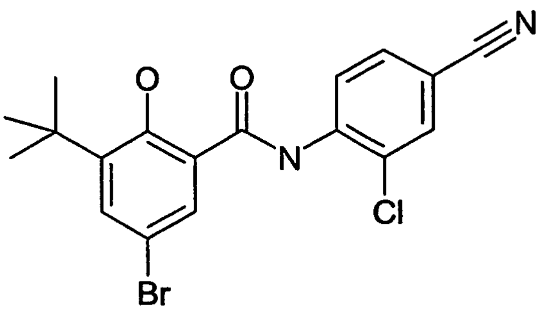

The title compound was prepared from 5-bromo-3-tert-butyl-2-hydroxybenzoic acid and 2-chloro-4-cyanoanilin.
$^1$H-NMR: (DMSO-$d_6$, 400 MHz): δ ppm 1.48 (s, 9 H) 7.51 (d, 1 H) 7.78 (d, 1 H) 7.92 (dd, 1 H) 8.19 (d, 1 H) 8.22 (d, 1 H) 10.88 (s, 1 H) 12.95 (s, 1H); HPLC-MS (Method A): *m*/*z* = 408 (M+); $R_t$ = 5.8 min.

**Example 95** (General procedure (D))

5-Bromo-3-tert-butyl-N-(4-cyanophenyl)-2-hydroxybenzamide.

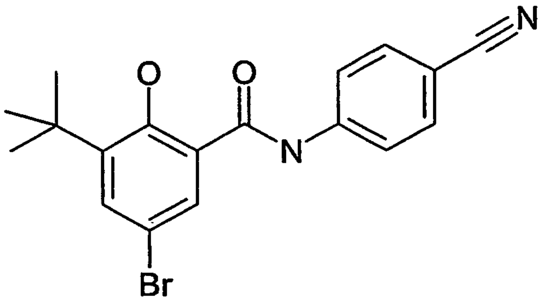

The title compound was prepared from 5-bromo-3-tert-butyl-2-hydroxybenzoic acid and 4-cyanoanilin.
$^1$H-NMR: (DMSO-$d_6$, 400 MHz): δ ppm 1.39 (s, 9 H) 7.49 (d, 1 H) 7.89 (m, 4 H) 8.19 (d, 1 H) 10.80 (s, 1 H) 12.83 (s, 1 H); HPLC-MS (Method A): *m*/*z* = 374 (M+1); $R_t$ = 5.7 min.

**Example 96** (General procedure (D))

3-Bromo-5-tert-butyl-6-hydroxy-2-methyl-N-(2-trifluoromethylphenyl)benzamide.

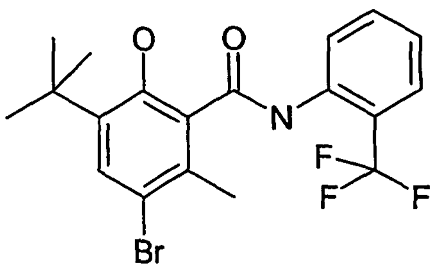

The title compound was prepared from 5-bromo-3-tert-butyl-6-methylsalicylic acid and 2-trifluoromethylanilin.
$^1$H-NMR: (CDCl3, 400 MHz): δ ppm 1.40 (s, 9 H) 2.57 (s, 3 H) 7.33 (dd, 1 H) 7.55 (s, 1 H) 7.65 (dd, 1 H) 7.69 (d, 1 H) 7.76 (br.s, 1H), 8.38 (d, 1 H) 9.45 (br.s, 1 H).

**Example 97** (General procedure (G))

2-(2-Bromo-1H-indol-3-ylmethylene)malononitrile

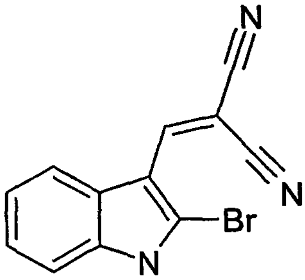

The title compound was prepared from 2-bromo-3-formylindol and malonitrile in ethanol using a catalytic amount of piperidine.
$^1$H NMR (DMSO-$d_6$): δ ppm 7.32 (m, 2 H) 7.49 (d, 1 H) 8.07 (m, 2 H) 13.75 (s, 1 H); HPLC-MS (Method A): *m*/*z* = 273 (M+1); $R_t$ = 3.60 min.

**Example 98** (General procedure (G))

2-(7-Bromo-2-methyl-1H-indol-3-ylmethylene)malononitrile

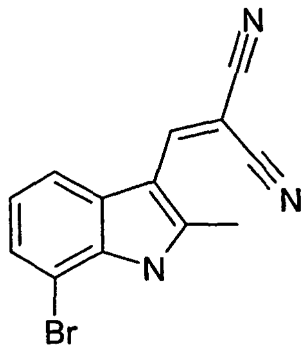

The title compound was prepared from 7-bromo-2-methyl-3-formylindol and malonitrile in ethanol using a catalytic amount of piperidine.
$^1$H NMR (DMSO-$d_6$): δ ppm 2.66 (s, 3 H) 7.18 (dd, 1 H) 7.48 (d, 1 H) 8.02 (d, 1 H) 8.37 (s, 1 H) 12.73 (br.s, 1 H)

**Example 99**(General procedure (G))

2-(5-Bromo-2-methyl-1H-indol-3-ylmethylene)malononitrile

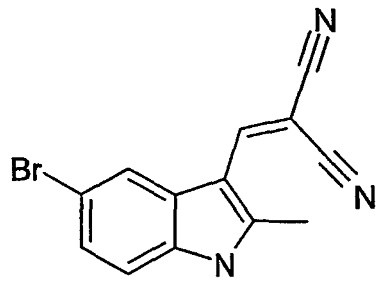

The title compound was prepared from 5-bromo-2-methyl-3-formylindol and malonitrile in ethanol using a catalytic amount of piperidine.
$^1$H NMR (DMSO-$d_6$): δ ppm 2.62 (s, 3 H) 7.60 (m, 2 H) 8.28 (s, 1 H) 8.31 (s, 1 H) 12.90 (br.s, 1H).

**PHARMACOLOGICAL METHODS**

Assay (I): Glucose utilisation in a human hepatocytes cell line (HEP-G2 cells)

**Assay description:**

The assay measures indirectly the activity of the respiratory chain in HEP-G2 cells by using D-(3-$^3$H(N))-glucose. The $^3$H-proton will first be released during the metabolism of glucose where it will be incorporated into water. The water is thereafter separated from the D-(3-$^3$H(N))-glucose by evaporation. Finally, the radioactivity in the water is determined using a Topcounter.

**Method:**

HEP-G2 cells obtained from ATCC (Maryland, USA), are cultured in assay medium (MEM medium) with the following addition 1x non-essential amino acids (M7145, 2 mM glutamine, 100 units/ml penicillin and streptomycin, 0.0075% sodium bicarbonate, 1 mM sodium pyrovate and 0.5% BSA (Bovin Serum Albumin, Sigma Missouri, USA)) at 37°C and 5% $CO_2$. All media are obtained by Gibco (Life Technologies, Maryland, USA) where not otherwise mentioned.

At day zero the cells are harvested using trypsin-EDTA and washed in assay medium using centrifugation. The cells are plated into single StripPlates wells (Coming B.V.Life Sciences, The Netherlands) that are placed into 24-well plates (Corning B.V.Life Sciences, The Netherlands) with a concentration of $2x10^4$ cells/100 μl/well. The cells are then incubated at 37°C and 5% $CO_2$ overnight.

The next day the compounds to be tested are diluted in DMSO (Sigma, Missouri, USA) to 100 times final concentration. They are then diluted to a final concentration in assay medium containing 10 μCi/ml D-(3-$^3$H(N))-glucose (PerkinElmer Life Sciences Inc.,Boston, USA). The medium is removed from the cells and 200 μl of the compound dilutions are added in duplicates. The cells are then incubated for another 3 hours at 37°C and 5% $CO_2$. Finally the cells are lysed by adding 50 μl 10% TCA (trichloroacetic acid). 300 μl of sterile water is then added to the 24-wells that surrounds the StripPlate wells. The plate is sealed with Top-seal-tape (Packard, PerkinElmer Life Sciences Inc.,Boston, USA) and the plate is incubated in a heating cupboard at 50°C to equilibrium the radioactive water formed in the respiratory chain into the water in the 24-well plate by evaporate. The plates incubate for 8 hours where the heating cupboard is turned off. The top seal is removed when the samples have reached room temperature. One ml scintillation liquid (Packard Microscient, PerkinElmer Life Sciences Inc.,Boston, USA) is added to all the samples and the radioactivity is determined using a Topcounter (Packard, PerkinElmer Life Sciences Inc.,Boston, USA). Non-specific activity is determined by evaporating 200 μl of the dilution medium containing the D-(3-$^3$H(N))-glucose into 300 μl sterile water, and total radioactivity is determined by counting 5 μl assay medium with 10 μCi/ml D-(3-$^3$H(N))-glucose.

**Calculations:**

The cpm (counts per minute) representing the non-specific radioactivity in the D-(3-$^3$H(N))-glucose is subtracted from all incubated values. Then mean basal value (samples without any compound added) is subtracted from the values of all stimulated samples (samples added different concentrations of the different test compounds). All these calculations are done using GraphPad Prism 3.0 (GraphPad software, Inc.). The maximal stimulation caused by the test compounds is calculated in percentage of maximal stimulation caused by either DNP or FCCP. If a test compound represents a saturated maximal stimulation that is less than 75% of maximal DNP/FCCP stimulation, that is, $E_{max}$ for the test compound is less than 75% of the $E_{max}$ for DNP or FCCP in Assay (I), it is considered as a <u>partial</u> stimulator, see figure 1, wherein the curve for SF6847 is compared to the curve for a partial stimulator according to the present invention. An $E_{max}$ of less than 75% of the $E_{max}$ for DNP or FCCP may signify that a saturation of the uncoupling process is taking place.

When calculating the concentration-response curve the maximal glucose utilisation stimulated by FCCP or DNP (subtracted non-specific and basal values) is converted to 100% and all the other values are expressed in percentage of this value. Using these percentage values and the compound concentration a concentration-response curve is calculated using sigmoidal dose-response (variable slope) equation (all the calculations are done by GraphPad Prism 3.0 (GraphPad software, Inc.));

<u>Equation 1</u>:

$$Y = Bottom + (Top - Bottom)/(1+10^{((LogEC50 - X)*slope)})$$

X is the logarithm of the molar concentration of the test compound.

Y is the degree of stimulation caused by the compound measured as a percentage of the maximal stimulation achieved by use of FCCP or DNP. Y starts at Bottom which is the value for the stimulation caused by the lowest concentration of the test compound and goes to Top which is the value for the stimulation caused by the highest concentration of the test compound and the curve for Y as a function of X has a sigmoid shape. The concentration of the test compound that stimulates the glucose utilisation with 50% is defined as $EC_{50}$. From the calculated $EC_{50}$ value, new concentrations of the compound corresponding to $3xEC_{50}$, $2x\ EC_{50}$, $EC_{50}/2$, $EC_{50}/3$ and control are tested in Assay (I). The results achieved with these concentrations are used to determine the slope in this area using the following equation:

**Equation 2:**

$$(Y_2-Y_0)/(Y_1-Y_0) = X^n$$

where $Y_0$ is the degree of stimulation measured as counts per minute (cpm) in Assay (I) in control samples without added test compound, $Y_1$ is the degree of stimulation measured as cpm in Assay (I) with added test compound in a concentration of either $EC_{50}/2$ or $EC_{50}/3$, and $Y_2$ is the degree of stimulation measured as cpm in Assay (I) with added test compound in concentration of either $2xEC_{50}$ or $3xEC_{50}$, X is either 2 or 3, and n is the slope.

The equation should be understood such that when $Y_1$ is the degree of stimulation with added test compound in a concentration of $EC_{50}/2$, then $Y_2$ is the degree of stimulation with added test compound in concentration of $2xEC_{50}$, and X is 2, and when $Y_1$ is the degree of stimulation with added test compound in a concentration of $EC_{50}/3$, then $Y_2$ is the degree of stimulation with added test compound in a concentration of $3xEC_{50}$, and X is 3.

The slope calculated by this equation is defined as the change in response divided with the change in test compound concentration around the $EC_{50}$ value of the compound. If this equation is high then a small change in test compound concentration cause a great change in the response, like when using DNP or FCCP. If the slope is close to unity then a small change in test compound concentration cause a small change in the response, a relation that can be used to determine that the test compound has a broader safety-window than DNP or FCCP. This slope may thus be used to describe safe chemical uncouplers for use according to the present invention (see figure 2).

Another way of describing safe chemical uncouplers is that no sign of toxicity (defined as a significant drop in glucose utilisation in Assay (I)) may occur within a ten fold increase of the concentration of the compound needed for achieving $E_{max}$. This characteristic will for the purpose of this application be designated a "long $E_{max}$", meaning that for compounds with a long $E_{max}$, the glucose utilisation does not decrease for concentrations ranging from the concentration of the compound needed for achieving $E_{max}$ to 10 times this concentration. Compounds with a long $E_{max}$ may be regarded as safe chemical uncouplers The following Table 1 shows the data from Assay (I) for the compounds of the examples.

**Equation 1:**

$$Y=Bottom + (Top - Bottom)/(1+10^{((LogEC50 - X)*slope)})$$

X is the logarithm of the molar concentration of the test compound.

Y is the degree of stimulation caused by the compound measured as a percentage of the maximal stimulation achieved by use of FCCP or DNP.

Y starts at Bottom which is the value for the stimulation caused by the lowest concentration of the test compound and goes to Top which is the value for the stimulation caused by the highest concentration of the test compound and the curve for Y as a function of X has a sigmoid shape. The concentration of the test compound that stimulates the glucose utilisation with 50% is defined as $EC_{50}$. From the calculated $EC_{50}$ value, new concentrations of the compound corresponding to $3xEC_{50}$, $2x EC_{50}$, $EC_{50}/2$, $EC_{50}/3$ and control are tested in Assay (I). The results achieved with these concentrations are used to determine the slope in this area using the following equation:

**Equation 2:**

$$(Y_2-Y_0)/(Y_1-Y_0) = X^n$$

where $Y_0$ is the degree of stimulation measured as counts per minute (cpm) in Assay (I) in control samples without added test compound, $Y_1$ is the degree of stimulation measured as cpm in Assay (I) with added test compound in a concentration of either $EC_{50}/2$ or $EC_{50}/3$, and $Y_2$ is the degree of stimulation measured as cpm in Assay (I) with added test compound in concentration of either $2xEC_{50}$ or $3xEC_{50}$, X is either 2 or 3, and n is the slope.

The equation should be understood such that when $Y_1$ is the degree of stimulation with added test compound in a concentration of $EC_{50}/2$, then $Y_2$ is the degree of stimulation with added test compound in concentration of $2xEC_{50}$, and X is 2, and when $Y_1$ is the degree of stimulation with added test compound in a concentration of $EC_{50}/3$, then $Y_2$ is the degree of stimulation with added test compound in a concentration of $3xEC_{50}$, and X is 3.

The slope calculated by this equation is defined as the change in response divided with the change in test compound concentration around the $EC_{50}$ value of the compound. If this equation is high then a small change in test

compound concentration cause a great change in the response, like when using DNP or FCCP. If the slope is close to unity then a small change in test compound concentration cause a small change in the response, a relation that can be used to determine that the test compound has a broader safety-window than DNP or FCCP. This slope may thus be used to describe safe chemical uncouplers for use according to the present invention (see figure 2).

Another way of describing safe chemical uncouplers is that no sign of toxicity (defined as a significant drop in glucose utilisation in Assay (I)) may occur within a ten fold increase of the concentration of the compound needed for achieving $E_{max}$. This characteristic will for the purpose of this application be designated a "long $E_{max}$", meaning that for compounds with a long $E_{max}$. the glucose utilisation does not decrease for concentrations ranging from the concentration of the compound needed for achieving $E_{max}$ to 10 times this concentration. Compounds with a long $E_{max}$ may be regarded as safe chemical uncouplers The following Table 1 shows the data from Assay (I) for the compounds of the examples.

Table 1

| **Example no.** | **FSK4 $EC_{50}$ (μM)** | **FSK4 $E_{max}$ (% of FCCP)** | **FSK4 Slope (% of FCCP)** | **Mito State-4** | **HEP-G2 $EC_{50}$ (μM)** | **HEP-G2 $E_{max}$ (% of FCCP)** | **HEP-G2 $E_{max}$ (μM)** | **HEP-G2 Slope** | **HEP-G2 Long $E_{max}$ / Tox** |
|---|---|---|---|---|---|---|---|---|---|
| 1 | | | | Ok | 40 | 45 | 100 | 1.5 | |
| 2 | | | | - | 290 | 35 | 1000 | | |
| 3 | | | | - | 270 | 75 | 1000 | | |
| 4 | | | | - | 150 | 70 | 1000 | 1.4 | |
| 5 | 0.1 | 100 | 115 | - | 0.06 | 95 | 0.3 | 1.7 | Long $E_{max}$ |
| 6 | | | | - | 1.6 | 95 | 10 | | Tox 0.1 mM |
| 7 | | | | | | | | | |
| 8 | | | | - | 12 | 80 | 100 | | |
| 9 | | | | - | 2.3 | 100 | 10 | 3.1 | Long $E_{max}$ |
| 10 | 0.3 | 80 | 95 | - | 0.37 | 100 | 3 | 1.7 | Long $E_{max}$ |
| 11 | 0.3 | 90 | 105 | | 0.7 | 75 | 3 | | Tox 0.03 mM |
| 12 | 0.3 | 85 | 170 | - | 1.2 | 70 | 10 | | Tox 0.03 mM |
| 13 | 1 | 85 | | - | 2.9 | 70 | 10 | | Tox 0.03 mM |
| 14 | 0.2 | 100 | 135 | - | 1.1 | 100 | 10 | | Tox 0.01 mM |
| 15 | 2 | 65 | 60 | - | 0.7 | 100 | 3 | | Long $E_{max}$ |
| 16 | 30 | 45 | | - | 11 | 100 | 100 | 2.3 | |
| 17 | | | | - | 6 | 45 | 30 | | Tox 0.1 m M |
| 18 | 6 | 100 | 50 | - | 0.6 | 80 | 3 | | Long $E_{max}$ |
| 19 | 1.1 | 55 | | - | 7 | 100 | 100 | 1.5 | |
| 20 | 26 | 90 | 80 | - | 50 | 55 | 300 | 1 | |
| 21 | | | | | | | | | |

(continued)

| Example no. | FSK4 $EC_{50}$ (μM) | FSK4 $E_{max}$ (% of FCCP) | FSK4 Slope (% of FCCP) | Mito State-4 | HEP-G2 $EC_{50}$ (μM) | HEP-G2 $E_{max}$ (% of FCCP) | HEP-G2 $E_{max}$ (μM) | HEP-G2 Slope | HEP-G2 Long $E_{max}$ / Tox |
|---|---|---|---|---|---|---|---|---|---|
| 22 | | | | - | 91 | 100 | 1000 | | |
| 23 | | | | - | 1.75 | 117 | 30 | | Long $E_{max}$ |
| 24 | | | | - | 42 | 65 | 100 | 2.3 | Long $E_{max}$ |
| 25 | 0.01 | 110 | 120 | Ok | 0.48 | 100 | 10 | 1.3 | Long $E_{max}$ |
| 26 | 90 | 95 | 120 | Ok | 225 | 100 | 500 | 3 | |
| 27 | 1 | 100 | 100 | Ok | 3 | 100 | 30 | 2.2 | Long $E_{max}$ |
| 28 | | | | - | 35.5 | 35 | 300 | | |
| 29 | | | | - | 13 | 65 | 30 | | Long $E_{max}$ |
| 30 | 50 | 100 | 85 | Ok | 19.5 | 60 | 100 | | Long $E_{max}$ |
| 31 | | | | - | 300 | 0 | | | |
| 32 | > 100 | > 15 | | - | 7 | 60 | 100 | | |
| 33 | | | | | | | | | |
| 34 | 0.4 | 90 | 95 | Ok | 1.4 | 80 | 10 | | Long $E_{max}$ |
| 35 | 5 | 50 | 90 | - | | | | | |
| 36 | 6.35 | 76 | 65 | - | | | | | |
| 37 | 0.03 | 110 | 95 | Ok | 0.035 | 100 | 1 | | Long $E_{max}$ |
| 38 | | | | - | 3.35 | 105 | 30 | | Long $E_{max}$ |
| 39 | | | | - | 6 | 55 | 10 | | Long $E_{max}$ |
| 40 | | | | Ok | 0.58 | 105 | 3 | | Long $E_{max}$ |
| 41 | | | | - | 4 | 103 | 30 | | Long $E_{max}$ |
| 42 | 1.1 | 75 | 120 | - | 3.3 | 89 | 10 | | Long $E_{max}$ |
| 43 | 0.3 | 80 | 75 | - | 0.85 | 95 | 3 | | Long $E_{max}$ |
| 44 | | | | - | 2 | 94 | 30 | | Long $E_{max}$ |
| 44 | 3.6 | 90 | 55 | - | 1.4 | 95 | 10 | | Long $E_{max}$ |

(continued)

| Example no. | FSK4 $EC_{50}$ (μM) | FSK4 $E_{max}$ (% of FCCP) | FSK4 Slope (% of FCCP) | Mito State-4 | HEP-G2 $EC_{50}$ (μM) | HEP-G2 $E_{max}$ (% of FCCP) | HEP-G2 $E_{max}$ (μM) | HEP-G2 Slope | HEP-G2 Long $E_{max}$ / Tox |
|---|---|---|---|---|---|---|---|---|---|
| 45 | | | | - | 0.6 | 96 | 3 | | Long $E_{max}$ |
| 46 | | | | - | 3 | 103 | 10 | | Long $E_{max}$ |
| 47 | 1 | 90 | 95 | - | 0.7 | 75 | 10 | | Tox 0.1 mM |
| 48 | 2.8 | 85 | 90 | - | 3.5 | 95 | 10 | | Long $E_{max}$ Tox 0.1mM |
| 49 | 0.3 | 95 | 95 | - | 0.95 | 105 | 3 | | Long $E_{max}$ |
| 50 | < 0.03 | 75 | 90 | Ok | 0.03 | 82 | 0.1 | | Long $E_{max}$ |
| 51 | 0.3 | 105 | 90 | - | 0.4 | 100 | 3 | | Long $E_{max}$ |
| 52 | 2.5 | 75 | 120 | - | 2.4 | 95 | 10 | | Long $E_{max}$ |
| 53 | 15 | 95 | 70 | - | 4.15 | 90 | 10 | | Long $E_{max}$ |
| 54 | 0.3 | 80 | 95 | - | 0.52 | 90 | 3 | | Long $E_{max}$ |
| 55 | 0.3 | 100 | 85 | - | 0.36 | 99 | 1 | | Long $E_{max}$ |
| 56 | 8 | 105 | 90 | Ok | 6.6 | 100 | 100 | | |
| 57 | | | | - | 0.31 | 90 | 10 | | Long $E_{max}$ |
| 58 | < 0.03 | 100 | 105 | Ok | 0.03 | 100 | 0.1 | | Long $E_{max}$ |
| 59 | 0.03 | 100 | 125 | - | 0.11 | 110 | 10 | | Long $E_{max}$ |
| 60 | 0.3 | 105 | 120 | - | 0.07 | 90 | 0.3 | | Long $E_{max}$ |
| 61 | 1.1 | 105 | 115 | - | 0.87 | 105 | 10 | | Long $E_{max}$ |
| 62 | 1.2 | 85 | 130 | - | 2.3 | 115 | 30 | | Long $E_{max}$ |
| 63 | 19 | 55 | 90 | - | 14 | 90 | 100 | | |
| 64 | | | | - | 2.6 | 105 | 10 | | Long $E_{ma}$x |
| 65 | | | | - | 5.3 | 100 | 10 | | Long $E_{max}$ |

(continued)

| Example no. | FSK4 $EC_{50}$ (μM) | FSK4 $E_{max}$ (% of FCCP) | FSK4 Slope (% of FCCP) | Mito State-4 | HEP-G2 $EC_{50}$ (μM) | HEP-G2 $E_{max}$ (% of FCCP) | HEP-G2 $E_{max}$ (μM) | HEP-G2 Slope | HEP-G2 Long $E_{max}$ / Tox |
|---|---|---|---|---|---|---|---|---|---|
| 66 | | | | - | 4.3 | 95 | 10 | | Long $E_{max}$ |
| 67 | | | | - | 5.9 | 85 | 30 | | Long $E_{max}$ |
| 69 | 9 | 40 | 110 | - | 100 | 58 | 300 | | Tox 1 mM |
| 70 | 7 | 180 | 85 | - | 32 | 112 | 300 | | |
| 71 | | | | - | 75 | 105 | 1000 | | |
| 72 | | | | Ok | 13 | 100 | 100 | | |
| 73 | 7 | 100 | 170 | - | 7.3 | 70 | 300 | | |
| 74 | 15 | 120 | 90 | - | 16 | 80 | 100 | | |
| 75 | 0.7 | 75 | 90 | Ok | 0.36 | 120 | 30 | | Tox 0.3 mM |
| 76 | 3.2 | 150 | 85 | - | 11 | 90 | 30 | | |
| 77 | 1 | 110 | 70 | Ok | 0.31 | 100 | 3 | | Long $E_{max}$ |
| 78 | | | | - | 2.9 | 80 | 10 | | Long $E_{max}$ |
| 79 | | | | - | 1 | 80 | 3 | | Long $E_{max}$ Tox |
| | | | | | | | | | 0.3 mM |
| 80 | 18 | 100 | 70 | - | 8 | 80 | 100 | | Tox 0.3 mM |
| 81 | 7 | 140 | 75 | - | 7 | 130 | 100 | | |
| 82 | 11 | 90 | 90 | - | 10 | 100 | 30 | | Long $E_{max}$ |
| 83 | 1.1 | 120 | 85 | - | | | | | |
| 84 | 1.4 | | 100 | | | | | | |
| 85 | 0.8 | | 49 | | | | | | |
| 86 | 1.0 | | 31 | | | | | | |
| 87 | 0.7 | | 41 | | | | | | |
| 88 | 0.5 | | 103 | | | | | | |
| 89 | 0.5 | | 84 | | | | | | |
| 90 | 0.6 | | 86 | | | | | | |
| 91 | 3.8 | | 47 | | | | | | |
| 92 | 1 | | 48 | | | | | | |
| 93 | 0.1 | | | | | | | | |
| 94 | 0.1 | 90 | 85 | - | - | - | - | | |
| 95 | - | - | - | - | - | - | - | | |

(continued)

| Example no. | FSK4 $EC_{50}$ (μM) | FSK4 $E_{max}$ (% of FCCP) | FSK4 Slope (% of FCCP) | Mito State-4 | HEP-G2 $EC_{50}$ (μM) | HEP-G2 $E_{max}$ (% of FCCP) | HEP-G2 $E_{max}$ (μM) | HEP-G2 Slope | HEP-G2 Long $E_{max}$ / Tox |
|---|---|---|---|---|---|---|---|---|---|
| 96 | 20 | 40 | - | - | - | - | - | | |
| 97 | 1.5 | 55 | 45 | - | - | - | - | | |
| 98 | 6.6 | 105 | 65 | - | - | - | - | | |
| 99 | 1.7 | 40 | - | - | - | - | - | | |

**Assay (II) - The effect of chemical uncouplers on mitochondrial respiration using isolated mitochondria.**

**Assay description:**

This assay is used to investigate if the increase in glucose utilisation caused by the test compounds observed in the glucose utilisation assay is due to an increase in the respiration of the mitochondria. This is done by measuring oxygen consumption in isolated mitochondria.

A Clark oxygen electrode is used to determine the oxygen consumption. The isolated mitochondria are added to medium containing oxygen and nutrients (e.g. Succinate) and the rate of oxygen consumptions is measured, when stabilized a small amount of ADP is added to increase the respiration and an increase in the rate of oxygen consumption is measured. When the rate of oxygen consumption again has stabilized the test compound is added and a further increase in oxygen consumption is seen. This experiment is done with and without the addition of oligomycin which is an inhibitor of the ATP-synthase. Finally, the test compound is examined without adding ADP. If the test compound stimulates the rate of oxygen consumption in all setups, it is regarded as a chemical uncoupler.

**Assay (III): Identification of chemical uncouplers (full or partial chemical uncouplers) that increase energy expenditure *in vivo***

The effect of the chemical uncouplers (full or partial agonists) on energy expenditure (oxygen consumption) in vivo is determined by indirect calorimetry. Briefly, animals are placed in airtight chambers. Air is continuously led to and from the chambers. The gas concentrations of oxygen ($O_2$) and carbondioxide ($CO_2$) in the air led to and from the chambers (inlet and outlet air) are recorded and the consumption of $O_2$ and the production of $CO_2$ are calculated. Based on the amount of $O_2$ consumed and $CO_2$ produced, energy expenditure is calculated. Compounds which at a given dose increase whole body energy expenditure without obvious deleterious effects are deemed to be chemical uncouplers that increase energy expenditure.

**Correlation between in vitro slope and in vivo saftey**

We have further shown that compounds that exhibit the preferred slopes *in vitro* in Assay (1) equation 2 also have a correspondingly low slope in the linear part of the dose-response curve in the in vivo EE assay (Assay III) compared to DNP.

Further more when the maximal tolerable dose (MTD) were determined for the compounds the relationship still exist; compounds with low *in vitro* slopes have a high safety window (defined as MTD/dose that activates a 20% increase in the EE in vivo).

**Assay (IV): Glucose utilisation in FSK-4 cells**

**Assay description:**

The assay measures indirectly the activity of the respiratory chain in FSK-4 cells by using D-(6-$^3$H(N))-glucose. The $^3$H-proton will first be released in the TCA cyclus and transported to the respiratory chain where it will be incorporated into water. The water is thereafter separated from the D-(6-$^3$H(N))-glucose by evaporation. Finally, the radioactivity in the water is determined using a Topcounter.

**Method:**

FSK-4 cells obtained from ATCC (Maryland, USA), are cultured in growth medium (McCoy's medium with the following addition 100 units/ml penicillin and streptomycin and 10 % FCS (fetal calf serum)) at 37°C and 5% $CO_2$. All media are obtained by Gibco (Life Technologies, Maryland, USA) where not otherwise mentioned.

At day zero the cells are harvested using trypsin-EDTA and washed in assay medium (MEM medium with the following addition 1x non-essential amino acids (M7145, 2 mM glutamin, 100 units/ml pencillin and streptomycin, 0.0075% sodium bicarbonate, 1 mM sodium pyrovate and 2 % horse serum) using centrifugation. The cells are plated into single StripPlates wells (Corning B.V.Life Sciences, The Netherlands) that are placed into 24-well plates (Coming B.V.Life Sciences, The Netherlands) with a concentration of $1{,}5 \times 10^4$ cells/100 μl assay medium/well. The cells are then incubated at 37°C and 5% $CO_2$ overnight.

The next day the compounds to be tested are diluted to different concentrations in DMSO (Sigma, Missouri, USA) to 100 times final concentration. They are then diluted to a final concentration in assay medium containing 10 μCi/ml D-(6-$^3$H(N))-glucose (PerkinElmer Life Sciences Inc.,Boston, USA). The medium is removed from the cells and 200 μl of the compound dilutions are added in duplicates. The cells are then incubated for another 24 hours at 37°C and 5% $CO_2$. Finally the cells are lysed by adding 50 μl 10% TCA (trichloroacetatic acid). 300 μl of sterile water is then added to the 24-wells that surrounds the StripPlate wells. The plate is sealed with Top-seal-tape (Packard, PerkinElmer Life Sciences Inc.,Boston, USA) and the plate is incubated in a heating cupboard at 50°C to equilibrium the radioactive water formed in the respiratory chain into the water in the 24-well plate by evaporate. The plates incubate for 8 hours where the heating cupboard is turned off. The top seal is removed when the samples have reached room temperature. One ml scintillation liquid (Packard Microscient, PerkinElmer Life Sciences Inc.,Boston, USA) is added to all the samples and the radioactivity is determined using a Topcounter (Packard, PerkinElmer Life Sciences Inc.,Boston, USA). Non-specific activity is determined by evaporating 200 μl of the dilution medium containing the D-(6-$^3$H(N))-glucose into 300 μl sterile water, and total radioactivity is determined by counting 5 μl assay medium with 10 μCi/ml D-(6-$^3$H(N))-glucose.

Calculations:

All calculations are done using GraphPad Prism 3.0 (GraphPad software, Inc.)

From concentration-response curves the half maximal concentration ($EC_{50}$) and maximal efficacy ($E_{max}$) are calculated using equation 1.

<u>Equation 1</u>:

$$Y=\text{Bottom} + (\text{Top} - \text{Bottom})/(1+10^{((\text{LogEC50} - X)*\text{slope})})$$

X is the logarithm of the molar concentration of the test compound.

Y is the degree of stimulation caused by the compound measured as percentage of the basal stimulation: Y starts at Bottom which is the value for the stimulation caused by the lowest concentration of the test compound and goes to Top which is the value for the stimulation caused by the highest concentration of the test compound and the curve for Y as a function of X has a sigmoid shape.

The calculated $EC_{50}$ value is then used to determine the concentrations used in a study where the Hill slope is calculated. In the Hill slope study concentrations between 0.05 to 10 times $EC_{50}$ are used.

From the double logarithmic plot of the increase in glucose utilisation and the compound concentration the Hill slope is calculated accordingly to equation 2.

**Equation 2:**

$$\frac{V}{V_{max}} = \frac{[X]^n}{K_H + [X]^n} \Leftrightarrow$$

$$\frac{V_{max}}{V} = \frac{K_H}{} + 1 \Leftrightarrow$$

$$\frac{V_{max} - V}{V} = \frac{K_H}{[X]^n} \Leftrightarrow$$

$$\mathrm{Log}\ V_{max} - \mathrm{Log}\ V = \mathrm{Log}\ K_H - n^*\mathrm{Log} \Leftrightarrow$$

$$\mathrm{Log}\ V = \mathrm{Log}\ V_{max} - \mathrm{Log}\ K_H + n^*\mathrm{Log}\ [X]$$

X is the molar concentration of the test compound
V is the increase in glucose utilisation
$V_{max}$ is the theoretic maximal no-limiting increase in glucose utilisation
$K_H$ is the Hill equation constant
n is the Hill slope

It is presumed, that the maximal increase in the glucose utilisation (as measured in the FSK-4 glucose utilisation assay) is limited by the capacity of the metabolism. It is furthermore presumed, that the glucose utilisation would reach a much higher degree of stimulation if the metabolism were not the limiting factor (the theoretical maximal stimulation in the FSK-4 glucose utilisation if metabolism were not the limiting factor is denoted $V_{max}$ in the equation above). Consequently, $V_{max}$- V $\approx V_{max}$, where V is the measured increase in FSK-4 glucose utilisation. Finally, it is assumed that Log $V_{max}$ -Log $K_H$ is a constant.

## Claims

1. The use of a compound of the general formula (III)

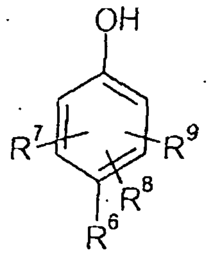

(III)

wherein
$R^6$ is halogen, -CHO, $-CO_2R^{43}$, $-COR^{43}$, $-SO_3H$, $-CCl_3$, $-CF_3$, -CN, $-CH=CH-R^{44}$, $-C(R^{44})(R^{45})$, $-SOR^{43}$, $-SO_2R^{43}$ or aryl substituted with from one to five substituents selected from halogen, -CHO, $-CO_2R^{43}$, $-COR^{43}$, $-SO_3H$, $-CCl_3$, $-CF_3$, -NO, $-NO_2$, -CN, $-CH=CH-R^{44}$, $-CH(R^{44})(R^{45})$, $-SOR^{43}$, $-SO_2R^{43}$. wherein
$R^{43}$ is hydrogen or alkyl; and
$R^{44}$ and $R^{45}$ independently of each other are halogen, -CHO, $-CO_2R^{46}$, $-COR^{46}$, $-SO_3H$, $-CCl_3$, $-CF_3$, -NO, $-NO_2$, -CN, $-SOR^{46}$, $-SO_2R^{46}$, wherein
$R^{46}$ is hydrogen, alkyl, or aryl;

$R^7$ is alkyl, halogen, alkyl-O-, alkyl-C(O)-, or alkyl-C(O)-O-; and
$R^8$ and $R^9$ independently of each other are hydrogen, alkyl, nitro, halogen, alkyl-O-, alkyl-C(O)-, alkyl-C(O)-O-, or aryl; or
$R^7$ and $R^8$ together form one of the diradicals

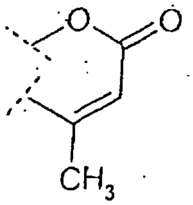 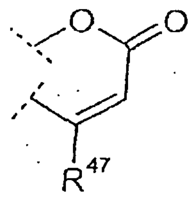 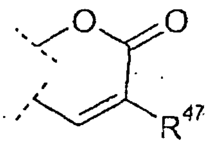 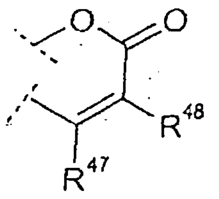,

wherein $R^{47}$ and $R^{48}$, independently of each other, are hydrogen, alkyl, nitro, halogen, alkyl-O-, alkyl-C(O)-, or alkyl-C(O)-O-,
wherein the two valence atoms in the diradical are attached to adjacent carbon atoms in the phenyl ring; and
$R^9$ is hydrogen, alkyl, nitro, halogen, alkyl-O-, or alkyl-C(O)-;
or a pharmaceutically acceptable salt, solvate or prodrug thereof,
for the manufacture of a medicament for treating a condition selected among: obesity, atherosclerosis, hypertension, diabetes, type 2 diabetes, impaired glucose tolerance, dyslipidemia, coronary heart disease, gallbladder disease, osteoarthritis, the aging process, damage to heart tissue, damage to endothelial cells, damage to neuronal tissue, Alzheimer's disease, cataract, diabetic microvascular diseases in the retina, renal glomerus and peripheral nerve cell apoptosis.

**2.** The use according to claim 1, wherein said condition is obesity.

**3.** The use according to claim 1, wherein said condition is type 2 diabetes.

**4.** The use according to claim 1, wherein said condition is dyslipidemia.

**5.** The use according to claim 3 or 4, wherein the patient having said condition is obese.

**6.** A compound of formula (III) as defined in claim 1 or a pharmaceutically acceptable salt or solvate thereof, for use in treating a condition as defined in any one of claims 1 to 5.

**7.** The use according to any one of claims 1 to 5 or the compound for use according to claim 6 wherein said compound is administered in combination with one or more further active substances.

**8.** A pharmaceutical composition for use in treating a condition as defined in any one of claims 1 to 5, said composition comprising a compound of formula (III) as defined in claim 1 or a pharmaceutically acceptable salt or solvate thereof, and one or more pharmaceutically acceptable carriers, excipients or diluents.

**9.** The pharmaceutical composition for use according to claim 8 wherein said composition additionally comprises one or more further active substances.

**Patentansprüche**

**1.** Verwendung einer Verbindung mit der allgemeinen Formel (III)

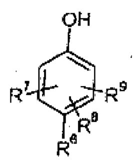

(III),

wobei
$R^6$ Halogen, -CHO, -$CO_2R^{43}$, -$COR^{43}$, -$SO_3H$, -$CCl_3$, -$CF_3$, -CN, -CH=CH-$R^{44}$, -C($R^{44}$)($R^{45}$), -$SOR^{43}$, -$SO_2R^{43}$ oder Aryl ist, das mit einem bis fünf Substituenten ausgewählt aus Halogen, -CHO, -$CO_2R^{43}$, -$COR^{43}$, -$SO_3H$, -$CCl_3$, -$CF_3$, -NO, -$NO_2$, -CN, -CH=CH-$R^{44}$, -CH($R^{44}$)($R^{45}$), -$SOR^{43}$, -$SO_2R^{43}$ substituiert ist, wobei
$R^{43}$ Wasserstoff oder Alkyl ist und
$R^{44}$ und $R^{45}$ unabhängig voneinander Halogen, -CHO, -$CO_2R^{46}$, -$COR^{46}$, -$SO_3H$, -$CCl_3$, -$CF_3$, -NO, -$NO_2$, -CN, -$SOR^{46}$, -$SO_2R^{46}$ sind, wobei
$R^{46}$ Wasserstoff, Alkyl oder Aryl ist,
$R^7$ Alkyl, Halogen, Alkyl-O-, Alkyl-C(O)- oder Alkyl-C(O)-O- ist und $R^8$ und $R^9$ unabhängig voneinander Halogen, Alkyl, Nitro, Halogen, Alkyl-O-, Alkyl-C(O)- oder Alkyl-C(O)-O- oder Aryl ist
oder
$R^7$ und $R^8$ zusammen eines der Diradikale

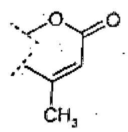 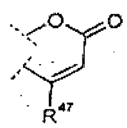 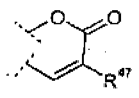 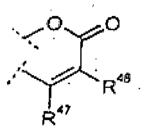

bilden, wobei $R^{47}$ und $R^{48}$ unabhängig voneinander Halogen, Alkyl, Nitro, Halogen, Alkyl-O-, Alkyl-C(O)- oder Alkyl-C(O)-O- sind,
wobei die beiden Valenzatome im Diradikal mit benachbarten Kohlenstoffatomen im Phenylring verknüpft sind und
$R^9$ Wasserstoff, Alkyl, Nitro, Halogen, Alkyl-O- oder Alkyl-C(O)- ist
oder eines pharmazeutisch akzeptablen Salzes, Solvats oder Prodrug davon zur Herstellung eines Medikaments zur Behandlung eines Zustands, der ausgewählt ist aus: Fettleibigkeit, Artheriosklerose, Hochdruck, Diabetes, Diabetes Typ 2, gestörte Glucosetoleranz, Dyslipidämie, koronare Herzerkrankung, Gallenblasenerkrankung, Osteoarthritis, Alterungsprozess, Schädigung von Herzgewebe, Schädigung von Endothelzellen, Schädigung von Nervengewebe, Alzheimer-Krankheit, Katarakt, diabetische Mikrogefäßschäden in der Retina, Apoptose am Nierenglomerulus und peripheren Nervenzellen.

**2.** Verwendung nach Anspruch 1, wobei der Zustand Fettleibigkeit ist.

**3.** Verwendung nach Anspruch 1, wobei der Zustand Diabetes Typ 2 ist.

**4.** Verwendung nach Anspruch 1, wobei der Zustand Dyslipidämie ist.

**5.** Verwendung nach Anspruch 3 oder 4, wobei der Patient mit dem Zustand ein Fettleibiger ist.

**6.** Verbindung mit der Formel (III), wie in Anspruch 1 definiert, oder ein pharmazeutisch akzeptables Salz oder Solvat davon zur Verwendung bei der Behandlung eines Zustands, wie in einem der Ansprüche 1 bis 5 definiert.

**7.** Verwendung nach einem der Ansprüche 1 bis 5 oder die Verbindung zur Verwendung nach Anspruch 6, wobei die Verbindung in Kombination mit einer oder mehreren weiteren Wirksubstanzen verabreicht wird.

**8.** Pharmazeutische Zusammensetzung zur Verwendung bei der Behandlung eines Zustands, wie in einem der Ansprüche 1 bis 5 definiert,
wobei die Zusammensetzung eine Verbindung mit der Formel (III), wie in Anspruch 1 definiert, oder ein pharmazeutisch akzeptables Salz oder Solvat davon und einen oder mehrere pharmazeutisch akzeptable Träger, Exzipienten oder Verdünnungsmittel umfasst.

**9.** Pharmazeutische Zusammensetzung zur Verwendung nach Anspruch 8, wobei die Zusammensetzung zusätzlich eine oder mehrere weitere Wirksubstanzen umfasst.

## Revendications

**1.** Utilisation d'un composé de formule générale (III)

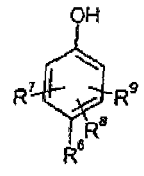

(III)

dans laquelle

$R^6$ est un atome d'halogène, -CHO, -$CO_2R^{43}$, -$COR^{43}$, -$SO_3H$, -$CCl_3$, -$CF_3$, -CN, -CH=CH-$R^{44}$, -C($R^{44}$)($R^{45}$), -$SOR^{43}$, -$SO_2R^{43}$ ou un groupe aryle substitué par un à cinq substituants choisies parmi un atome d'halogène, -CHO, -$CO_2R^{43}$, -$COR^{43}$, -$SO_3H$, -$CCl_3$, -$CF_3$, -NO, -$NO_2$, -CN, -CH=CH-$R^{44}$, -CH($R^{44}$)($R^{45}$), -$SOR^{43}$, -$SO_2R^{43}$, où

$R^{43}$ est un atome d'hydrogène ou un groupe alkyle ; et

$R^{44}$ et $R^{45}$ indépendamment l'un de l'autre, sont un atome d'halogène, -CHO, -$CO_2R^{46}$, -$COR^{46}$, -$SO_3H$, -$CCl_3$, -$CF_3$, -NO, -

$NO_2$, -CN, -$SOR^{46}$, -$SO_2R^{46}$, où

$R^{46}$ est un atome d'hydrogène, un groupe alkyle, ou aryle ;

$R^7$ est un groupe alkyle, un atome d'halogène, un groupe alkyl-O-, alkyl-C(O)-, ou alkyl-C(O)-O- ; et

$R^8$ et $R^9$ indépendamment l'un de l'autre, sont un atome d'hydrogène, un groupe alkyle, nitro, un atome d'halogène, un groupe alkyl-O-, alkyl-C(O)-, alkyl-C(O)-O-, ou aryle ;

ou

$R^7$ et $R^8$ forment ensemble l'un des diradicaux

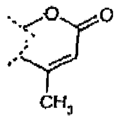 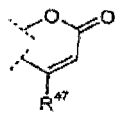 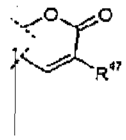 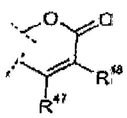

où $R^{47}$ et $R^{48}$, indépendamment l'un de l'autre, sont un atome d'hydrogène, un groupe alkyle, nitro, un atome d'halogène, un groupe alkyl-O-, alkyl-C(O)-, ou alkyl-C(O)-O-,

où les deux atomes de valence dans le diradical sont attachés à des atomes de carbone adjacents dans le cycle phényle; et $R^9$ est un atome d'hydrogène, un groupe alkyle, nitro, un atome d'halogène, un groupe alkyl-O- ou alkyl-C(O)- ;

ou un sel, solvate ou promédicament pharmaceutiquement acceptable de celui-ci,

pour la fabrication d'un médicament destiné au traitement d'une affection choisie parmi : l'obésité, l'athérosclérose, hypertension, le diabète, le diabète de type 2, une dégradation de la tolérance au glucose, la dyslipidémie, une maladie coronarienne, une maladie vésiculaire, l'arthrose, le processus de vieillissement, les dommages infligés au tissu cardiaque, les dommages infligés aux cellules endothéliales, les dommages infligés au tissu neuronal, la maladie d'Alzheimer, la cataracte, les maladies microvasculaires liées au diabète dans la rétine, le glomérule rénal et l'apoptose des cellules nerveuses périphériques.

**2.** Utilisation selon la revendication 1, dans laquelle ladite affection est l'obésité.

**3.** Utilisation selon la revendication 1, dans laquelle ladite affection est le diabète de type 2.

**4.** Utilisation selon la revendication 1, dans laquelle ladite affection est la dyslipidémie.

**5.** Utilisation selon la revendication 3 ou 4, dans laquelle le patient atteint de ladite affection est obèse.

**6.** Composé de formule (III) tel que défini dans la revendication 1 ou sel ou solvate pharmaeeutiquement acceptable de celui-ci, pour une utilisation dans le traitement d'une affection telle que définie dans l'une quelconque des revendications 1 à 5,

**7.** Utilisation selon l'une quelconque des revendications 1 à 5 ou composé pour une utilisation selon la revendication

6, où ledit composé est administré en combinaison avec une ou plusieurs substances actives supplémentaires.

**8.** Composition pharmaceutique pour une utilisation dans le traitement d'une affection telle que définie dans l'une quelconque des revendications 1 à 5, ladite composition comprenant un composé de formule (III) tel que défini dans la revendication 1 ou un sel ou solvate pharmaceutiquement acceptable de celui-ci, et un ou plusieurs supports, excipients ou diluants pharmaceutiquement acceptables.

**9.** Composition pharmaceutique pour une utilisation selon la revendication 8, dans laquelle ladite composition comprend également une ou plusieurs substances actives supplémentaires.

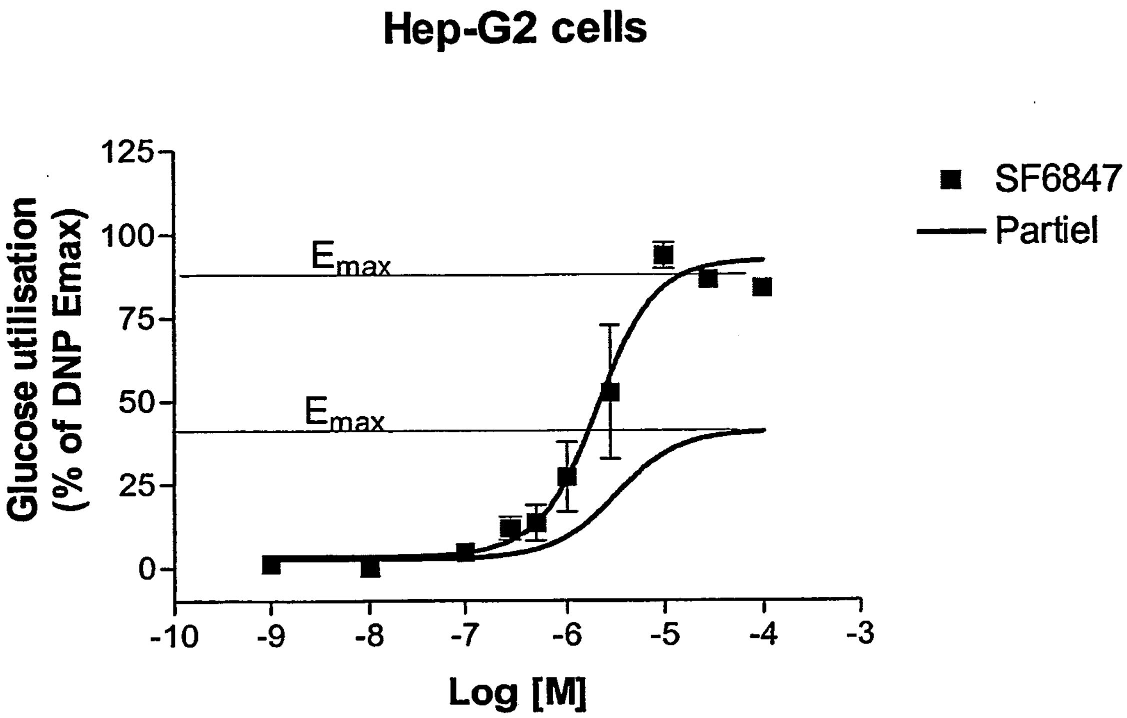
Hep-G2 cells
Glucose utilisation (% of DNP Emax)
125
100
75
50
25
0
Emax
Emax
-10
-9
-8
-7
-6
-5
-4
-3
Log [M]
SF6847
Partiel

Fig. 1

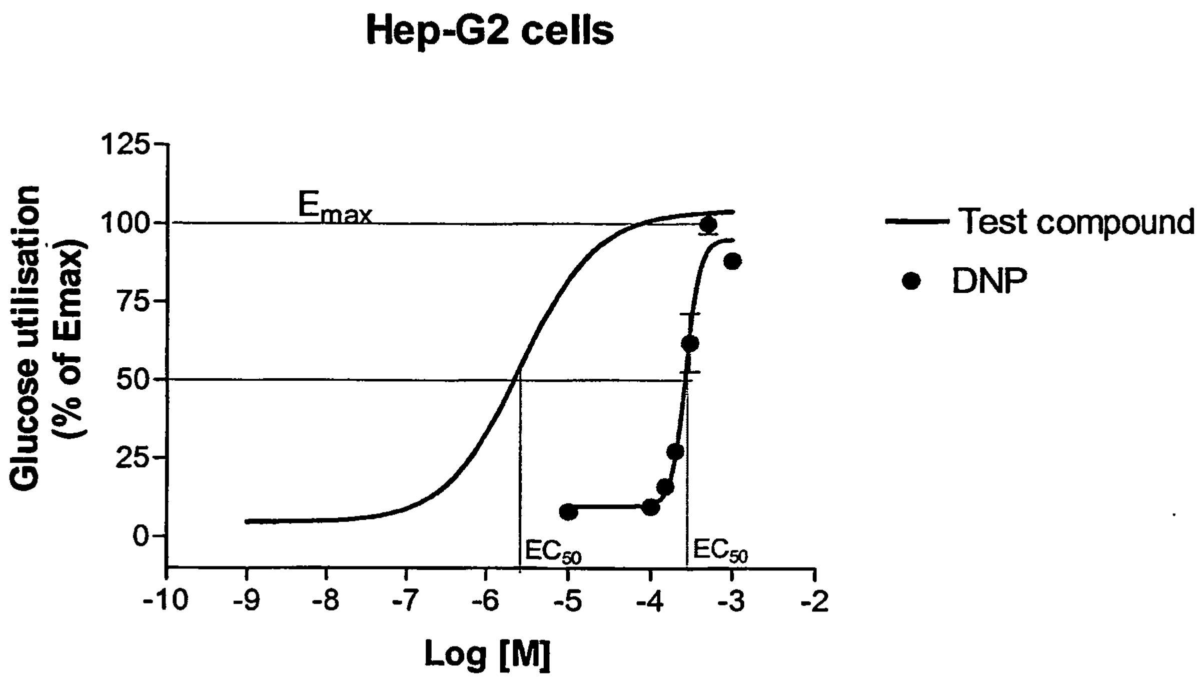
Hep-G2 cells
Glucose utilisation (% of Emax)
125
100
75
50
25
0
Emax
EC50
EC50
-10
-9
-8
-7
-6
-5
-4
-3
-2
Log [M]
Test compound
DNP

**Fig. 2**

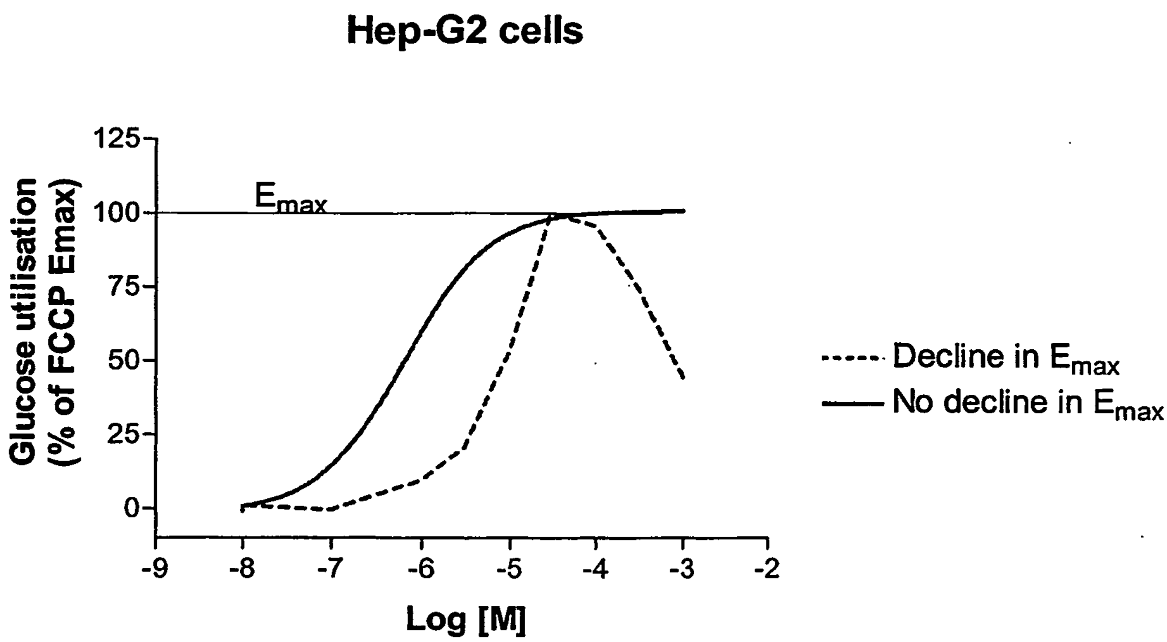
Hep-G2 cells
Glucose utilisation
(% of FCCP Emax)
125
100
75
50
25
0
Emax
-9
-8
-7
-6
-5
-4
-3
-2
Log [M]
Decline in Emax
No decline in Emax

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0006143 A
- US 4673691 A, Bachynsky
- WO 9808871 A
- WO 9726265 A
- WO 9903861 A
- WO 0037474 A
- WO 9901423 A
- WO 0039088 A
- WO 0042026 A
- WO 0208209 A, Hoffmann La Roche
- WO 9741097 A
- WO 9741119 A
- WO 9741120 A
- WO 0041121 A
- WO 9845292 A
- WO 9919313 A
- WO 0050414 A
- WO 0063191 A
- WO 0063192 A
- WO 0063193 A
- WO 0023425 A
- WO 0023415 A
- WO 0023451 A
- WO 0023445 A
- WO 0023417 A
- WO 0023416 A
- WO 0063153 A
- WO 0063196 A
- WO 0063209 A
- WO 0063190 A
- WO 0063189 A
- WO 9709040 A
- WO 00142023 A
- WO 0063208 A
- WO 0064884 A
- US 4356108 A
- US 4166452 A
- US 4265874 A


### Non-patent literature cited in the description


- **De Grey et al.** *Eur J. Biochem,* 2002, vol. 269, 1995 ff
- **Miyoshi H et al.** Quantitative releationship between protenophoric and uncoupling activities of analogs of SF6847 (2,6-di-t-butyl-4-(2',2'-dicyanovinyl)phenol). *Biochimica et Biophysica Acta,* 1987, vol. 891, 293-299
- **Terada H et al.** Structural Requirements of Salicylanilides for Uncoupling Activity in Mitochondria Quantitative Analysis of Structure- Uncoupling Relationships. *Biochimica et Biophysica Acta,* 1988, vol. 936, 504-512
- **Goto K et al.** *Chem. Pharm. Bull.,* 1996, vol. 44 (3), 547-551
- **T. Shimokawa et al.** *Drug Development Research,* 2000, vol. 51 (1), 43-48
- **Batterham et al.** *Nature,* 2002, vol. 418, 650-654
- Remington: The Science and Practice of Pharmacy. Mack Publishing Co, 1995
- Remington: The Science and Practice of Pharmacy. Mack Publishing Co, 2000
- *J. Pharm. Sci.,* 1977, vol. 66, 2